(19) Europäisches Patentamt / European Patent Office / Office européen des brevets

(11) **EP 4 192 613 B1**

(12) # EUROPEAN PATENT SPECIFICATION

(45) Date of publication and mention of the grant of the patent: **06.05.2026 Bulletin 2026/19**

(21) Application number: **21755943.4**

(22) Date of filing: **05.08.2021**

(51) International Patent Classification (IPC):
***B01J 13/10*** *(2006.01)* ***A01N 25/28*** *(2006.01)*
***A23P 10/30*** *(2016.01)* ***A61K 8/11*** *(2006.01)*
***A61K 9/50*** *(2006.01)* ***C11D 3/50*** *(2006.01)*

(52) Cooperative Patent Classification (CPC):
**B01J 13/10; A01N 25/28; A23P 10/30; A61K 8/11; A61K 8/25; A61K 8/585; A61K 8/733; A61K 8/736; A61K 8/922; A61K 8/9789; A61Q 19/00;**
A61K 2800/10

(86) International application number:
**PCT/EP2021/071890**

(87) International publication number:
**WO 2022/029238 (10.02.2022 Gazette 2022/06)**

(54) **HYBRID CORE-SHELL MICROCAPSULES FOR ENCAPSULATING ACTIVE INGREDIENTS**

HYBRIDE KERN-SCHALE-MIKROKAPSELN ZUR VERKAPSELUNG VON WIRKSTOFFEN

MICROCAPSULES HYBRIDES À NOYAU/COQUILLE POUR L’ENCAPSULATION DE PRINCIPES ACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2020 EP 20382731**

(43) Date of publication of application:
**14.06.2023 Bulletin 2023/24**

(73) Proprietors:
- **Fundació Eurecat**
**08290 Cerdanyola del Vallès (ES)**
- **Creaciones Aromáticas Industriales, S.A.**
**08192 Sant Quirze Del Vallès (ES)**

(72) Inventors:
- **TYLKOWSKI, Bartosz**
**43004 TARRAGONA (ES)**
- **MONTORNÉS DAURA, Josep Maria**
**43002 TARRAGONA (ES)**
- **GARCIA VALLS, Ricard**
**43893 ALTAFULLA (ES)**
- **HAPONSKA, Monika**
**43007 TARRAGONA (ES)**
- **MARIA COTA, Iuliana**
**43001 TARRAGONA (ES)**
- **MARTINEZ CHAMORRO, Denia**
**08192 SANT QUIRZE DEL VALLÈS (ES)**
- **BERNAL LABRADOR, Cristobal**
**08028 BARCELONA (ES)**
- **PUIGPINOS COLILLAS, Albert**
**08740 SANT ANDREU DE LA BARCA (ES)**

(74) Representative: **ZBM Patents - Zea, Barlocci & Markvardsen**
**Rambla de Catalunya, 123**
**08008 Barcelona (ES)**

(56) References cited:
**WO-A1-2017/161364**
**WO-A1-2020/131866**
**CN-A- 109 135 260**
**CN-A- 109 438 638**
**CN-A- 110 498 952**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **FAN Q ET AL: "Preparation of organic-inorganic double shell microcapsule system used for controlling temperature of hydrophobic small molecules involves preparing mixture including silica precursor, adding to chitosan solution, sonicating, and stirring", WPI / 2017 CLARIVATE ANALYTICS,, vol. 2015, no. 79, 12 August 2015 (2015-08-12), XP002801356**
- **LEE W J: "Microencapsulated mosquito repellent composition comprises core material contains mosquito repellent agent, wall material contains biodegradable polymer, inorganic binder and silane coupling agent, and binder, which is safe", WPI / 2017 CLARIVATE ANALYTICS,, vol. 2018, no. 34, 27 April 2018 (2018-04-27), XP002801357**
- **WANG D: "Multi-functional anti-mosquito polyvinyl chloride material contains polyvinyl chloride cross-linked resin, stabilizer, lubricant, chelating agent, plasticizer, mosquito repellent microcapsules, coupling agent and modifier", WPI / 2017 CLARIVATE ANALYTICS,, vol. 2017, no. 26, 4 January 2017 (2017-01-04), XP002801358**
- **CHEN B ET AL: "Microcapsule type flame retardant composition comprises anionic emulsifier, glucomannan, triaryl phosphate, phenolic resin, aminomethylphosphonic acid, coupling agent, flame retardant synergist and baicalin", WPI / 2017 CLARIVATE ANALYTICS,, vol. 2018, no. 35, 20 April 2018 (2018-04-20), XP002801359**
- **LIAO Y ET AL: "Micro-capsule slow-release agent slurry comprises starch glue slurry, modified pigment and micro-capsule slow-release crosslinking agent, where starch cement comprises starch, a polymer binder, and water", WPI / 2017 CLARIVATE ANALYTICS,, vol. 2020, no. 1, 11 February 2020 (2020-02-11), XP002801360**
- **CHEN M ET AL: "Preparing insulin biobased releasable vesicle macromolecules involves utilizing sodium alginate and chitosan oligosaccharide based on biological macromolecules group, processing sodium alginate and chitosan oligosaccharide", WPI / 2017 CLARIVATE ANALYTICS,, vol. 2016, no. 31, 25 November 2015 (2015-11-25), XP002801361**
- **DATABASE WPI Week 201579, Derwent World Patents Index; AN 2015-58996M, XP002801356**
- **DATABASE WPI Week 201834, Derwent World Patents Index; AN 2018-362530, XP002801357**
- **DATABASE WPI Week 201726, Derwent World Patents Index; AN 2017-04719Y, XP002801358**
- **DATABASE WPI Week 201835, Derwent World Patents Index; AN 2018-32759E, XP002801359**
- **DATABASE WPI Week 202001, Derwent World Patents Index; AN 2020-16278W, XP002801360**
- **DATABASE WPI Week 201631, Derwent World Patents Index; AN 2015-81080M, XP002801361**

## Description

### Technical Field

The present invention relates to the field of chemistry, more particularly to encapsulation of active ingredients. In particular, the invention relates to hybrid inorganic-organic core-shell microcapsules, and to a process for their preparation. It also relates to compositions comprising the hybrid microcapsules, and their use as disinfectants as well as for the delivery of active ingredients.

### Background Art

Microencapsulation technology provides material barriers (shell) between bioactive materials (core), such as essential oils, fragrance, aroma, etc. and the environment, therefore allowing improvement of their stability in final products and during processing. Recently, it became the scope of interest of numerous studies due to the wide range of possible applications for encapsulated products (i.e.: in food industry, medical sector, cosmetics, textiles).

Depending on the application, microcapsules preparation methods and conditions need to be adjusted to obtain the particles with specific size, shell thickness and permeability.

Biopolymers, due to their high capacity of functionalization, are promising materials for the production of the microcapsule wall. Nevertheless, recent studies put a lot of attention to the biodegradability of materials used for this purpose.

The patent application CN104825421 discloses organic-inorganic double-shell microcapsules which encapsulate hydrophobic small-molecules which are prepared by 1) mixing an amphiphilic triblock copolymer, a silica precursor, a silane coupling agent, and a hydrophobic small molecule in an organic solvent; 2) dissolving the chitosan in an acid solution; and 3) adding 1) dropwise into 2).

The patent application WO2017015885 discloses microcapsules containing: (i) a microcapsule core having an active material, and (ii) a microcapsule wall formed of a first polymer and second polymer. The first polymer is a sol-gel polymer. The second polymer is gum arabic, purity gum ultra, gelatin, chitosan, xanthan gum, plant gum, carboxymethyl cellulose, sodium carboxymethyl guar gum, or a combination thereof.

On the other hand, alginate-based microcapsules have been reported as being used for encapsulating active ingredients. However, these capsules generally show mechanical instability and high porosity which make them unsuitable for practical applications. To overcome this problem, a polycation layer has traditionally been added to the alginate gels. However, the microcapsules obtained do not show an appropriate balance between enough mechanical stability which allows their storage until use, controlled and progressive release of the active ingredients, and biodegradability of the capsule materials.

Thus, there is still a need of providing further solutions for encapsulating active ingredients which overcome the problems of the prior art.

### Summary of Invention

The present inventors have developed new hybrid organic-inorganic core-shell microcapsules containing active ingredients (e.g. essential oils) in liquid form which show high encapsulation efficiency and excellent mechanical stability and low leakage. In particular, the inventors have found that when a coupling agent, particularly an organosilane comprising at least a quaternary ammonium group, a hydroxy group or a carboxylate group, is incorporated to the network forming the shell which comprises a cationic polymer (e.g. chitosan), and anionic polymer (e.g. alginate), and a silicate, compact microcapsules are obtained with reduced leakage of the encapsulated materials, unlike microcapsules which do not include the coupling agent. The reduced leakage increases safety to the user and non-target organisms, and also allows using reduced amounts of active ingredients.

Without being bound to theory it is thought that the ability of the coupling agent to form covalent bonds with the silicate network on the one hand, and ionic bonds with the organic polymeric materials on the other hand, gives rise to an improved distribution of the inorganic structure through the shell network, which in turn results in a well-integrated and compacted shell.

Furthermore, the microcapsules of the invention are able to release the encapsulated active ingredients (for example by friction or by a pH change) in a controlled and progressive way, while resulting in non-toxic and non-contaminant materials.

A further advantage of the microcapsules of the invention is that different active ingredients of hydrophilic nature (i.e. water-soluble) and/or of lipophilic nature (i.e. oil-soluble) may be encapsulated in the same capsule as aqueous core material or oil in water nanoemulsion core material.

Therefore, a first aspect of the invention relates to organic-inorganic core-shell microcapsules having a particle

size distribution D90 from 2 to 800 μm, wherein:

a) the shell of each microcapsule forms a network which comprises:

i) an anionic polymer which comprises a plurality of carboxylate or sulfonate groups,
ii) a cationic polymer which comprises a plurality of quaternary ammonium groups,
iii) an alkali metal silicate or a silicate precursor, and
iv) a coupling agent which is an organosilane comprising at least a terminal group selected from a quaternary ammonium group, hydroxy, and carboxylate, and
v) a cross-linking agent which is a multivalent metallic cation, and

b) the core of each microcapsule comprises one or more active ingredients in liquid form.

The microcapsules of the invention may be prepared by complex coacervation techniques. Thus, a second aspect of the invention relates to a process for the preparation of the hybrid core-shell microcapsules as previously defined, which comprises the following steps:

a) providing an aqueous solution comprising a polymer which comprises a plurality of carboxylic acid or sulfonic acid groups; an alkali metal silicate or a silicate precursor; a coupling agent which is an organosilane comprising at least a terminal group selected from amino, hydroxy, and carboxylic acid; and one or more active ingredients to obtain mixture A, wherein in solution the polymer is in anionic form comprising a plurality of carboxylate or sulfonate groups, and the terminal group of the coupling agent is in the form of a quaternary ammonium group, hydroxy, or carboxylate group;
b) dissolving a polymer which comprises a plurality of amino groups in an acidic aqueous solution and adding a cross-linker to obtain solution B, wherein in solution the polymer is in cationic form comprising a plurality of quaternary ammonium groups; and
c) dropping mixture A onto solution B to obtain the microcapsules.

The capsules of the present invention may be used in a number of applications in the food, textile, perfumery, cosmetic, home-care, personal-care, air-care, surface-care, animal-care, agricultural and pharmaceutical industries alone or forming part of a composition including further components. Thus, it also forms part of the invention a composition comprising the organic-inorganic core-shell microcapsules as defined herein, and one or more excipients or carriers.

A further aspect of the invention relates to the use of the organic-inorganic core-shell microcapsules or a composition comprising them as a disinfectant.

A further aspect of the invention relates to the use of the organic-inorganic core-shell microcapsules or a composition comprising them for the delivery of active ingredients such as the delivery of biopesticides, perfumes, biologically active compounds, or any other soluble material in the active material forming capsule core.

**Brief Description of Drawings**

FIG. 1 is a schematic view representing the microcapsule inner structure (network formed by organic and inorganic components) of an embodiment of the invention, where the filled circles represent the anionic polymer (in this embodiment a polymer having carboxylate groups) and the triangles represent the cationic polymer (in this embodiment a polymer having quaternary ammonium groups). The figure shows the interactions between the organic components and a cross-linker (also shown in FIG. 2), the interactions of the inorganic components (also shown in FIG. 3), and the interactions between the coupling agent (in this case a coupling agent having a quaternary ammonium terminal group connected to the silicon atom by an aliphatic chain), and the anionic polymer (empty ovals).

FIG. 2 is a schematic view representing the organic part of the structure depicted in FIG. 1 showing the interactions between the anionic polymer and a multivalent cation (cross-linker, in this embodiment $Ca^{2+}$), and between the anionic polymer and the cationic polymer.

FIG. 3 is a schematic view representing the inorganic part of the structure depicted in FIG.1 showing the links between the metasilicate banana structure formed by the silicate (in this embodiment sodium silicate) and the coupling agent.

FIG. 4 shows an OM image (Optical Microscope image) at magnification of 40X of the microcapsule of example 2 after being stored for 4 months at 22 °C.

FIG. 5 shows an OM image at magnification of 40X of the microcapsule of example 5 after being stored for 4 months at 22 °C.

FIG. 6 shows an OM image at magnification of 40X of the microcapsule of comparative example 10 after being stored for 4 months at 22 °C.

FIG. 7 shows the elemental analysis carried out by Energy-dispersive X-ray spectroscopy with Environmental Scanning Electron Microscope (ESEM) confirming the presence of silica in the shell of the microcapsules of Example 1 at 1.7 KeV.

FIG. 8 shows the RAMAN spectra of the microcapsules of Example 1. The peak at the wavenumber of 477 $cm^{-1}$ corresponds to the Si-O-Si bond.

**Detailed description of the invention**

All terms as used herein in this application, unless otherwise stated, shall be understood in their ordinary meaning as known in the art. Other more specific definitions for certain terms as used in the present application are as set forth below and are intended to apply throughout the description and claims.

The term "about" or "around" as used herein refers to a range of values $\pm$ 10% of a specified value. For example, the expression "about 10" or "around 10" includes $\pm$ 10% of 10, i.e. from 9 to 11.

Unless otherwise stated, all percentages mentioned herein are expressed in weight with respect to the total weight of the microcapsules or the microcapsule shell, provided that the sum of the amounts of the components is equal to 100% content.

For the purposes of the invention, the term "hybrid" microcapsule as used herein refers to a microcapsules which comprises organic and inorganic constituents.

The term "core-shell microcapsule" also referred herein simply as "microcapsule" or "capsule" refers to physical entity generally spherical which comprises two differentiated phases: the shell, also referred to as wall, coating, membrane or external phase, which comprises a network or matrix formed at least by the polymer materials such as alginate and chitosan, the silicate or a precursor of it, and the coupling agent; and the core, also referred to as nucleus or internal phase, which comprises one or more active hydrophilic and/or lipophilic or active ingredients in liquid form. The core is encapsulated by the shell. The use of a microcapsule allows the creation of a physical barrier between the shell and the core and thus may protect sensitive ingredients (e.g. flavours and essential oils) from the external medium (moisture, pH, oxidation, etc.).

The microcapsules of the invention typically have an average particle size ranging from 1 to 1000 $\mu$m and can be obtained by a complex coacervation process. The average particle size can be determined in several different ways, for example using an Image-J software and the optical micrographs of the microcapsules captured at room temperature, by DeltaPix Invenio 3S digital camera connected with Microscope "Axiovert" 40C for transmitted-light brightfield and phase contrast with condenser 0.4, inclusive object traverser M. The microcapsules size is in a range between 5 and 100 microns and the average microcapsules size is 40 microns.

The expression "the shell forms a network" refers to the fact that the components of the shell, in particular the polymer materials such as alginate and chitosan, the silicate or a precursor of it, the coupling agent and optionally the cross-linker form a three-dimensional structure in which the different chemical entities are interconnected to one another unlike a structure in which some components are merely coated by other components.

For the purposes of the invention, the term "coupling agent" refers to a bifunctional compound which is able to form chemical bonds with the silicate through its silane part by covalent -O-Si-O- bonds, and to the polymeric materials (e.g. alginate/chitosan) through its quaternary ammonium/hydroxy/carboxylate part by ionic bonds.

The term "biodegradable", as used herein refers to the fact that the polymers are degraded within a period that is acceptable in the desired application, typically less than five years and most preferably less 30 days, once exposed to the biodegradability tests described by the Organisation for Economic Cooperation and Development (OECD) Guideline for testing of chemicals, number 301 Adopted: 17.07.92.

The term "hydrophilic" or "water-soluble" active ingredient as used herein refers to an active ingredient which can be dissolved in any amount of water. The term "hydrophobic", "lipophilic" or "oil-soluble" active ingredient as used herein refers to an active ingredient which can be dissolved in any amount of oil.

The terms "cross-linker" or "cross-linking agent" refers to a molecule or part of a molecule which is able the form chemical bonds (e.g. covalent or ionic) which another compound, in particular with one of the polymers of the shell.

For the purposes of the invention, the term "surfactant" refers to a material which lowers the surface tension of a liquid and the interfacial tension between two liquids.

The term "acidic aqueous solution" refers to a solution having an acid pH, i.e., a pH below 7, more particularly a pH

from 2 to 4.

The term -($C_1$-$C_{12}$)alkyl refers to a saturated branched or linear hydrocarbon chain which contains from 1 to 12 carbon atoms and only single bonds. Non-limiting examples of -($C_1$-$C_{12}$)alkyl groups include methyl, ethyl, propyl, isopropyl, butyl, isobutyl, sec-butyl, tert-butyl, pentyl, or hexyl. The term -($C_1$-$C_{12}$)alkyl encompasses the groups -($C_1$-$C_6$)alkyl and -($C_1$-$C_3$)alkyl. The term -O($C_1$-$C_{12}$)alkyl refers to an alkoxy group, wherein the alkyl part is as previously defined. Non-limiting examples of -O($C_1$-$C_n$)alkyl groups include methoxy, ethoxy, propoxy, isopropoxy, butoxy, or isobutoxy. The term -O($C_1$-$C_{12}$)alkyl encompasses the groups -O($C_1$-$C_6$)alkyl and -O($C_1$-$C_3$)alkyl.

The term "($C_6$-$C_{12}$)aryl" as used herein refers to an aromatic group which contains from 6 to 12 carbon atoms. Non-limiting examples of "($C_6$-$C_{12}$)aryl include phenyl, naphthyl, and biphenyl.

A halogen substituent means fluoro, chloro, bromo or iodo.

The expression "one or more" as used herein referred to active ingredients, excipients or carriers, substituents, atoms, groups, surfactants or cross-linkers means that there can be one or more of such entities, in particular 1, 2, 3 or 4.

<u>Anionic polymers</u>

The term "anionic polymer" as used herein refers to a polymer carrying a net negative charge, in particularly comprising a plurality of carboxylate or sulfonate groups. The anionic polymer is obtained when a polymer comprising a plurality of carboxylic acid or sulfonic acid groups is placed in an aqueous solution, in particular at about pH 4, and the carboxylic acid or sulfonic acid groups are deprotonated. The anionic polymer may additionally comprise other negatively charged species attached along the polymer chain.

In one embodiment, optionally in combination with one or more features of the various embodiments described above or below, the anionic polymer is soluble in water at pH from 2 to 6.

In one embodiment, optionally in combination with one or more features of the various embodiments described above or below, the anionic polymer is selected form the group consisting of alginate, carrageenan, gellan gum, carboxyl methyl cellulose, hyaluronic acid, and a combination thereof. More particularly, the anionic polymer is alginate.

<u>Alginate</u>

Alginic acid is a biocompatible polysaccharide distributed widely in the cell walls of brown algae. When forming salts with metals such as sodium and calcium, it is referred to alginate. It is a linear copolymer with homopolymeric blocks of (1-4)-linked β-D-mannuronate (M) and its C-5 epimer α-L-guluronate (G) residues, respectively, covalently linked together in different sequences or blocks. The monomers may appear in homopolymeric blocks of consecutive G-residues (G-blocks), consecutive M-residues (M-blocks) or alternating M and G-residues (MG-blocks). Its chemical structure corresponds to the following formula:

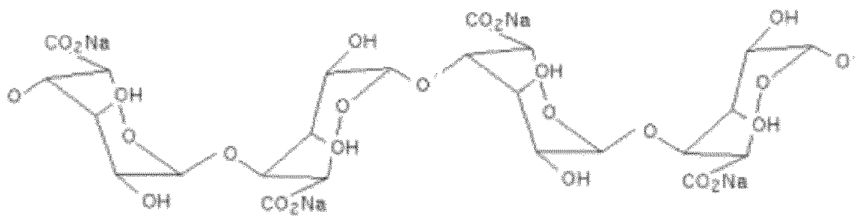

Alginates may be characterized by its molecular weight (MW) and the ratio between M and G blocks (M/G ratio). The M/G ratio can be assessed by Fourier Transform InfraRed Spectroscopy (FTIR). The molecular weight may be determined by size exclusion chromatography, light scattering or by measuring the intrinsic viscosity of sonicated solutions.

In one embodiment, optionally in combination with one or more features of the various embodiments described above or below, the alginate has a M/G ratio from 65/35 to 85/15, more particularly about 70/30.

In one embodiment, optionally in combination with one or more features of the various embodiments described above or below, the alginate has an average molecular weight from 10 to 500 kg/mol, more particularly from 60 to 380 kg/mol, and even more particularly from 100 to 300 kg/mol.

<u>Cationic polymers</u>

The term "cationic polymer" as used herein refers to a polymer carrying a net positive charge, in particularly comprising a plurality of quaternary ammonium groups. The cationic polymer is obtained when a polymer comprising a plurality of amino groups is placed in an acidic aqueous solution, and the amino groups are protonated.

In one embodiment, optionally in combination with one or more features of the various embodiments described above or below, the cationic polymer is soluble in water at pH from 2 to 5.

In one embodiment, optionally in combination with one or more features of the various embodiments described above or below, the cationic polymer is selected form the group consisting of chitosan, N,O-carboxymethyl-chitosan, N,O-glycolic-chitosan, poly-L-lysine, and a combination thereof. More particularly, the anionic polymer is chitosan.

Chitosan

Chitosan is a biodegradable and biocompatible linear polysacharide present in the shell of all crustaceans (crab, prawns, shrimp, spider crabs, lobsters, squid and krill) and also in the cell wall of insects, worms and fungi. It has strong antibacterial properties as well as low toxicity. Chitosan is composed of randomly distributed β-(1-4)-linked D-glucosamine and N-acetyl-D-glucosamine units and has the following formula:

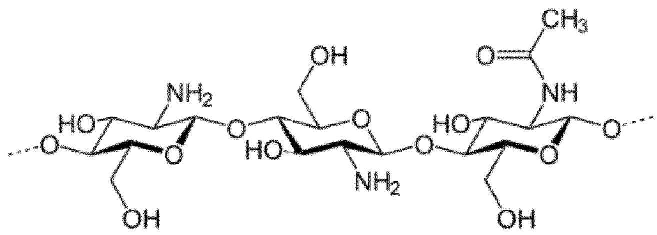

Chitosan is able to form a polyion complex with anionic polymers such as alginate through its quaternary ammonium groups. It is obtained from N-deacetylation of chitin. The degree of acetylation (DA) of a given chitosan is the percentage of units N-acetyl-D-glucosamine with respect to the total number of units (N-acetyl-D-glucosamine units and D-glucosamine units) constituting this chitosan. The degree of acetylation allows distinguishing between chitin and chitosan. Thus, chitosan typically has a degree of acetylation below 50%.

The degree of acetylation may be determined by different methods well-known in the art. The hydrolysis of chitosan by acids such as sulfuric acid or oxalic acid liberates acetyl groups. The acetic acid produced can be determined by (HPLC) with a spectrophotometric or photodiode array detector (PDA), and by Fourier Transform InfraRed Spectroscopy with Attenuated Total Reflection (FTIR-ATR).

In one embodiment, optionally in combination with one or more features of the various embodiments described above or below, the chitosan has a DA of less than 40%, more particularly less than 30%, and even more particularly less than 20%.

Chitosan typically has an average molecular weight in the range of 100 to 800 kg/mol. Molecular weight may be determined by size exclusion chromatography, light scattering or by measuring the intrinsic viscosity of sonicated solutions.

In one embodiment, optionally in combination with one or more features of the various embodiments described above or below, the chitosan has an average molecular weight from 100 to 350 kg/mol, more particularly from 125 to 275 kg/mol and even more particularly from 140 to 250 kg/mol.

Silicates and precursors thereof

The alkali metal silicate or a silicate precursor is used to form an inorganic net in the wall (shell) structure. Non-limiting examples of alkali metal silicate include lithium silicate, sodium silicate or potassium silicate.

For the purposes of the invention, the term "silicate precursor" refers to a compound which contains silicon and oxygen which is capable of forming a silicate structure by methods well-known in the art such as e.g. condensation. Non-limiting examples of silicate precursors include, tetramethoxysilane (TMOS), tetraethoxysilane (TEOS), tetrapropoxysilane (TPOS), methyldiethoxysilane (MDES), 3-(glycidoxypropyl)trimethoxysilane (GPTMS), 3-(glycidoxypropyl) triethoxysilane (GPTES), tetrakis(2-hydroxyethyl) orthosilicate (THEOS), methyl-trimethoxysilane (MTMS), methyltriethoxysilane (MTES), ethyltrimethoxysilane (ETMS), ethyltriethoxysilane (ETES), propyltrimethoxysilane (PTMS), propyltriethoxysilane (PTES), butyltrimethoxysilane (BTMS), butyltriethoxysilane (BTES), isopropyltrimethoxysilane (IPTMS), isopropyltriethoxysilane (IPTES), phenyltriethoxysilane, phenyltrimethoxysilane, vinyltrimethoxysilane (VTMS), vinyltriethoxysilane (VTES), allyltrimethoxysilane, allyltriethoxysilane, 3- chloropropyltriethoxysilane or methacryloyloxypropyltrimethoxysilane.

In one embodiment, optionally in combination with one or more features of the various embodiments described above or below, the shell comprises an alkali metal silicate. More particularly the alkali metal silicate is selected from the group consisting of lithium silicate, sodium silicate and potassium silicate, and even more particularly the alkali metal

silicate is sodium silicate.

In one embodiment, optionally in combination with one or more features of the various embodiments described above or below, the shell comprises a silicate precursor, more particularly a silicate precursor has the formula $Si(R^1)_m(R^2)_n$, wherein:

m and n are an integer from 1 to 3 provided that n+m is 4;
each $R^1$ is independently halogen, hydroxy or $-O(C_1-C_{12})$alkyl; and
each $R^2$ is independently selected from halogen, $-(C_1-C_{12})$alkyl, vinyl, allyl, phenyl,
and $-O(C_1-C_{12})$alkyl; wherein $-(C_1-C_{12})$alkyl, and $-O(C_1-C_{12})$alkyl are unsubstituted or substituted with one or more substituents selected from the group consisting of halogen, hydroxy, glycicyl and methacryloyl.

In another embodiment, optionally in combination with one or more features of the various embodiments described above or below, the silicate precursor has the formula $Si(R^1)_m(R^2)_n$, wherein each $R^1$ and each $R^2$ is independently $-O(C_1-C_{12})$alkyl unsubstituted or substituted as previously defined, more particularly each $R^1$ and each of $R^2$
are $-O(C_1-C_6)$alkyl, even more particularly each $R^1$ and each of $R^2$ are methoxy (i.e. the silicate precursor is TMOS), or alternatively each $R^1$ and each of $R^2$ are ethoxy (i.e. the silicate precursor is TEOS), or alternatively each $R^1$ and each of $R^2$ are propoxy (i.e. the silicate precursor is TPOS). Even more particularly the silane used as a silica precursor is TEOS.

The coupling agent

As mentioned above, the coupling agent is an organosilane comprising at least a terminal group selected from quaternary ammonium ($-NH_3^+$, $-NH_2^+R^6$), hydroxy (-OH), and carboxylate ($-COO^-$) in solution. Thus, the organosilane may be an aminosilane, an hydroxysilane or a carboxylatesilane, respectively. Aminosilanes are capable to form ionic bonds through their quaternary ammonium groups with the carboxylate or sulfonate groups present in the anionic polymer. Hydroxysilanes and carboxylatesilanes are capable to form ionic bonds through their hydroxyl carboxylate groups with the protonated quaternary ammonium groups present in the cationic polymers.

Non-limiting examples of available aminosilanes include 3-aminopropyltrimethoxysilane (APTMS), 3-aminopropyltriethoxysilane (APTES), 3-(2-aminoethylamino)propyltrimethoxysilane (AAPTS), (3-aminopropyl)dimethylethoxysilane (APMES), (3-aminopropyl)-diethoxymethylsilane (APDEMS), 3-[2-(2-aminoethylamino)ethylamino]propyltrimethoxysilane, or trimethoxy[3-(methylamino)propyl]silane (MAPTMS).

Non-limiting examples of available carboxylatesilanes include 4-(trimethoxysilyl)butanoic acid, 4-(triethoxysilyl) butanoic acid, 5-(trimethoxysilyl)pentanoic acid, 5-(triethoxysilyl)-pentanoic acid, 10-(trimethoxysilyl)decanoic acid, or 10-(triethoxysilyl)decanoic acid.

Hydroxysilanes and carboxylatesilanes may be obtained by processes well-known in the field, for example as described in the papers Patrocinio A.F. et al., J. Chem. Soc., Perkin Trans. 1, 1999, pp. 3133-3137, Tachibana Y. et al., Silicon 2012, 4, pp. 167-174, and Feinle A. et al., Chem. Commun. 2015, 51, pp. 2339-2341.

In one embodiment, optionally in combination with one or more features of the various embodiments described above or below, the coupling agent is is an organosilane of the formula $Si(R^3)_o(R^4)_p$, wherein:

o and p are an integer from 1 to 3 provided that o+p is 4;
each $R^3$ is independently halogen, hydroxy or $-O(C_1-C_{12})$alkyl; and
each $R^4$ has independently one of the formulas below:

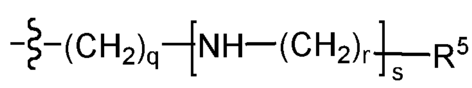

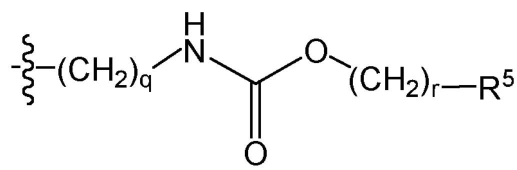

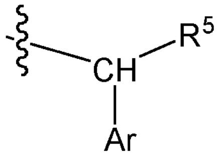

wherein q is an integer from 1 to 5, r is an integer from 1 to 5, s is an integer from 0 to 2, Ar is unsubstituted $(C_6-C_{12})$aryl or $(C_6-C_{12})$aryl substituted with one or more substituents selected from the group consisting of halogen, hydroxy, $-(C_1-C_3)$alkyl, $-(C_1-C_3)$alkyl substituted with one or more halogen atoms, $-O(C_1-C_3)$alkyl, and $-O(C_1-C_3)$alkyl substituted with one or more halogen atoms, and

$R^5$ is selected from $-NH_3^+$, $-NH_2^+R^6$, hydroxy and carboxylate ($-COO^-$), and $R^6$ is hydrogen or $-(C_1-C_6)$alkyl.

In one embodiment, optionally in combination with one or more features of the various embodiments described above or below, o is 3, and each $R^3$ is $-O(C_1-C_{12})$alkyl, more particularly $-O(C_1-C_6)$alkyl, and even more particularly ethoxy or methoxy.

In one embodiment, optionally in combination with one or more features of the various embodiments described above or below, p is 1.

In one embodiment, optionally in combination with one or more features of the various embodiments described above or below, in $R^4$, s is 0 and q is from 1 to 9, more particularly from 1 to 6, or from 1 to 5, or from 1 to 4, and even more particularly from 1 to 3.

In one embodiment, optionally in combination with one or more features of the various embodiments described above or below, in $R^4$, s is 2, r is 2, and q is from 1 to 9, more particularly from 1 to 6, and even more particularly from 1 to 3.

In one embodiment, optionally in combination with one or more features of the various embodiments described above or below, in $R^4$, s is 1, r is 2, and q is from 1 to 9, more particularly from 1 to 6, and even more particularly from 1 to 3.

In one embodiment, optionally in combination with one or more features of the various embodiments described above or below, o is 3; each $R^3$ is $-O(C_1-C_{12})$alkyl, more particularly $-O(C_1-C_6)$alkyl; p is 1; s is 0; and q is from 1 to 9, more particularly from 1 to 6, or from 1 to 5, or from 1 to 4, and even more particularly from 1 to 3.

In one embodiment, optionally in combination with one or more features of the various embodiments described above or below, $R^5$ is $-NH_3^+$, $-NH_2^+R^6$, more particularly $-NH_3^+$.

In one embodiment, optionally in combination with one or more features of the various embodiments described above or below, $R^5$ is hydroxy.

In one embodiment, optionally in combination with one or more features of the various embodiments described above or below, $R^5$ is carboxylate.

In one embodiment, optionally in combination with one or more features of the various embodiments described above or below, the coupling agent is selected from the group consisting of 3-aminopropyltrimethoxysilane (APTMS), 3-aminopropyltriethoxysilane (APTES), 3-(2-aminoethylamino)propyltrimethoxysilane (AAPTS), (3-aminopropyl)-dimethylethoxysilane (APMES), (3-aminopropyl)diethoxymethylsilane (APDEMS), 3-[2-(2-aminoethylamino)ethylamino]propyltrimethoxysilane, trimethoxy[3-(methylamino)-propyl]silane (MAPTMS), 4-(trimethoxysilyl)butanoic acid, 4-(triethoxysilyl)butanoic acid, 5-(trimethoxysilyl)pentanoic acid, 5-(triethoxysilyl)-pentanoic acid, 10-(trimethoxysilyl)-decanoic acid, and 10-(triethoxysilyl)decanoic acid. More particularly, the coupling agent is APTES or 5-(triethoxysilyl)pentanoic acid, even more particularly, APTES.

Cross-linking agents

The microcapsules of the invention comprise one or more cross-inkers in the shell as further components also forming part of the network.

The use of cross-linking agents may improve the mechanical strength of the microcapsule by interacting with the polymers of the microcapsule, in particular with the negatively charged groups of the anionic polymer. Multivalent metallic cations, and particularly divalent cations, in the form of a salt are used as cross-linking agents. Non-limiting examples of cross-linking agents which are used include multivalent metallic cations and particularly divalent cations such as salts of calcium ($Ca^{2+}$), magnesium ($Mg^{2+}$), copper ($Cu^{2+}$), barium ($Ba^{2+}$), beryllium ($Be^{2+}$), chromium ($Cr^{2+}$), cobalt ($Co^{2+}$), iron ($Fe^{2+}$), niquel ($Ni^{2+}$), and zinc ($Zn^{2+}$). Any salts of these cations may be used provided that it is soluble in an aqueous acidic solution. Some non-limiting examples of salts of these cations include chloride, phosphate, carbonate, oxalate, or sulfate.

According to the invention, the shell of the microcapsule further comprises a cross-linking agent. The cross-linking agent is a multivalent metallic cation, and more particularly a divalent cation, even more particularly a divalent cations selected from the group consisting of $Ca^{2+}$, $Mg^{2+}$, $Cu^{2+}$, $Ba^{2+}$, $Be^{2+}$, $Cr^{2+}$, $Co^{2+}$, $Fe^{2+}$, and $Zn^{2+}$.

In an embodiment, optionally in combination with one or more features of the various embodiments described above or below, the shell of the microcapsule further comprises a divalent cation selected from the group consisting of

$Ca^{2+}$, $Mg^{2+}$, and $Cu^{2+}$, more particularly a cation selected from the group consisting of $Ca^{2+}$, $Mg^{2+}$, and $Cu^{2+}$, in the form of a chloride, phosphate, carbonate, oxalate, or sulfate salt.

In one embodiment, optionally in combination with one or more features of the various embodiments described above or below, the shell of the microcapsule further comprises one or more cross-linking agents; and the anionic polymer, the cationic polymer, the alkali metal silicate or a silicate precursor, the coupling agent, and the cross-linking agents form a three-dimensional structure in which these components are interconnected to one another.

In one embodiment, optionally in combination with one or more features of the various embodiments described above or below, in the disclosed microcapsules, the cross-linking agents are bonded to the anionic polymer through ionic bonds.

## Active ingredients

The capsules of the present invention comprise one or more active ingredients in the core of the microcapsule and may be used in a number of applications in the food, textile, perfumery, cosmetic, home-care, personal-care, air-care, surface-care, animal-care, agricultural and pharmaceutical industries.

The encapsulated active ingredients are in liquid form. An advantage of the microcapsules of the invention is that different active ingredients may be encapsulated in the same capsule. Thus, the active ingredients may be of hydrophilic nature (i.e. water-soluble) and/or of lipophilic nature (i.e. oil-soluble). When the microcapsule only comprises hydrophilic active ingredients, they may be present in the form of an aqueous solution. When the microcapsule additionally comprises lipophilic active ingredients the active ingredients may be present in the form of an emulsion such as for example an oil-in-water (o/w) emulsion, in particular in a ratio oil to water from 1 to 10, more particularly from 1 to 2.

In one embodiment, optionally in combination with one or more features of the various embodiments described above or below, the core of the microcapsule of the invention comprises one or more active hydrophilic active ingredients and one or more active hydrophobic active ingredients.

Some non-limiting examples of active agents which can be encapsulated in the microcapsules include hydrophilic perfume raw materials, essential oils, hydrophobic polyphenols and their derivatives, hydrophilic aqueous and/or alcoholic extracts obtained from plant materials, hydrophilic and hydrophobic probiotics, insect repellents, insect sex pheromones, plant growth promoters, fertilizers, fungicides, probiotics, enzymes, biocides, drugs, as well as biologically active compounds which could be extracted from plants or pomace.

The term "essential oil" is used herein to refer to liquid, volatile and lipophilic substances with aromatic properties. In numerous studies, essential oils have shown antibacterial, antifungal, antiparasitic as well as insecticidal and antioxidant properties. Some examples of essential oils which can be encapsulated in the capsules of the invention include, without limitation, mint, spearmint, citronella, rosemary, oregano, and tea tree oil.

When a lipophilic active ingredient is to be encapsulated in the microcapsules of the invention the use of a surfactant may be advantageous because it may further reduce the leakage of active ingredients. Examples of surfactant agents include, without limitation, non-ionic surfactants, cationic surfactants, amphoteric surfactants, zwitterionic surfactants, and mixtures thereof. Of particular interest are non-ionic surfactants, such as for example polyoxyethylene (20) sorbitan monooleate (Tween 80®), polyoxyethylene (20) sorbitan monolaurate (Tween 20®), polyoxyethylene (2) cetylether (Brij 52®), polyoxyethylene (10) cetylether (Brij 56®), or polyoxyethylene (20) cetylether (Brij 58®)

In one embodiment, optionally in combination with one or more features of the various embodiments described above or below, the microcapsule of the invention comprises a surfactant. In a more particular embodiment, the organic-inorganic core-shell microcapsule comprises polyoxyethylene (20) sorbitan monooleate.

For the intended application the microcapsules may be used alone or forming part of a composition including further components. Thus, it also forms part of the invention a composition comprising the organic-inorganic core-shell microcapsules as defined herein, and one or more excipients or carriers.

The compositions comprising the microcapsules of the invention may be for example pharmaceutical compositions, cosmetic compositions or agricultural compositions.

Thus, in one embodiment, optionally in combination with one or more features of the various embodiments described above or below, the disclosure relates to a pharmaceutical, cosmetic or agricultural composition comprising the organic-inorganic core-shell microcapsule as defined herein, in particular an effective amount of the microcapsule, together with appropriate pharmaceutically, cosmetically or agriculturally acceptable excipients or carriers.

The term "home care" includes excipients and/or carriers which can be incorporated a liquid laundry detergent composition, a dish washer composition, a fabric softening composition, a bath-room cleaning composition, or an all-purpose home cleaning composition.

The term "surface-care" includes excipients and/or carriers which can be incorporated a liquid or spray or emulsion of a hard surface cleaning composition, or an all-purpose cleaning composition.

The term "personal-care" includes excipients and/or carriers which are suitable to be used in compositions with personal care products involving bar and liquid soaps, body washes, shampoos, conditioners, liquid and/or solid

detergents, and the like.

The term "air-care" includes the devices and/or excipients and/or carriers which are suitable to be used in composition for air refresher.

The term "pharmaceutically acceptable" refers to excipients and/or carriers which are suitable to be used in compositions with medical indications.

The term "animal-care" includes the excipients and/or carriers which be contacted with animal skin or/and animal hair/coat without undue toxicity, incompatibility, instability, allergic response, among others.

The term "cosmetically acceptable" refers to excipients and/or carriers which can be contacted with skin without undue toxicity, incompatibility, instability, allergic response, among others.

The term "agriculturally acceptable carrier" as used herein refers to a carrier that is not unacceptably damaging to a plant or its environment, and/or not unsafe to the user or others that may be exposed to the material when used as described herein.

The term "effective amount" refers to the amount of encapsulated active agents which provide the intended therapeutic, cosmetic or agricultural effect upon its application.

Examples of carriers include, without limitation solvents such as water, alcohols such ethanol or isopropanol, glycols such as propylene glycol or butylene glycol, and silicones and waxes; adjuvants, dispersants, surfactants, binders, or stabilizers.

In one embodiment, optionally in combination with one or more features of the various embodiments described above or below, the microcapsules of the invention or compositions comprising them may be used as disinfectants products of wide application in hospitals, nursing homes, public transport, etc. Thus, a further aspect of the invention relates to the use of the organic-inorganic core-shell microcapsule or a composition comprising it as defined herein as disinfectants.

Disinfectant compositions comprising the microcapsules as defined herein also form part of the disclosure. In one embodiment, optionally in combination with one or more features of the various embodiments described above or below, the composition comprising the microcapsules as defined herein is a disinfectant composition, more particularly a disinfectant composition selected form the group consisting of household products, sanitizers, liquid or powder laundry detergents, liquid dish detergents, all-purpose cleaners including bucket dilutable cleaners, toilet cleaners, bathroom cleaners, room deodorizers, floor and window cleaners, furniture cleaners, or air fresheners.

The organic-inorganic core-shell microcapsules of the invention may also be used for delivering active ingredients such as agricultural products. Some non-limiting examples of active ingredients include insect repellents, insect sex pheromones, plant growth promoters, fertilizers, fungicides, probiotics, enzymes, biocides, drugs, as well as biologically active compounds which could be extracted from plants or pomace. Accordingly, a further aspect of the invention relates to the use of the organic-inorganic core-shell microcapsules or a composition comprising them for the delivery of active ingredients.

In one embodiment, optionally in combination with one or more features of the various embodiments described above or below, the composition comprising the organic-inorganic core-shell microcapsule is a liquid or spray composition.

<u>The hybrid core-shell microcapsules</u>

As mentioned above the first aspect of the invention relates to organic-inorganic core-shell microcapsules having a particle size distribution D90 from 2 to 800 $\mu$m, wherein:

a) the shell of each microcapsule forms a network which comprises:

i) an anionic polymer which comprises a plurality of carboxylate or sulfonate groups,
ii) a cationic polymer which comprises a plurality of quaternary ammonium groups,
iii) an alkali metal silicate or a silicate precursor, and
iv) a coupling agent which is an organosilane comprising at least a terminal group selected from a quaternary ammonium group, hydroxy, and carboxylate, and
v) a cross-linking agent which is a multivalent metallic cation, and

b) the core of each microcapsule comprises one or more active ingredients in liquid form.

In one embodiment, optionally in combination with one or more features of the various embodiments described above or below, in the microcapsules of the invention, the anionic polymer, the cationic polymer, the alkali metal silicate or a silicate precursor and the coupling agent form a three-dimensional structure in which the anionic polymer, the cationic polymer, the alkali metal silicate (e.g. sodium silicate or potassium silicate) or a silicate precursor, and the coupling agent are interconnected to one another.

In one embodiment, optionally in combination with one or more features of the various embodiments described above or below, in the microcapsules of the invention, the anionic polymer is bonded to the cationic polymer by ionic bonds.
In one embodiment, optionally in combination with one or more features of the various embodiments described above or below, in the microcapsules of the invention, the alkali metal silicate (e.g. sodium or potassium silicate) or a silicate precursor can form a metasilicate banana structure or/and other structures such as: polyhedral oligomeric silsesquioxanes, open cage, ladder structure, random structure, spherosilicate. Such silicate structures are disclosed for example in - Issa A. A. et al, Polymers 2019, 11, pp. 537.
In one embodiment, optionally in combination with one or more features of the various embodiments described above or below, in the microcapsules of the invention, the alkali metal silicate is bonded to the coupling agent by covalent -O-Si-O- bonds.
In one embodiment, optionally in combination with one or more features of the various embodiments described above or below, in the microcapsules of the invention, the coupling agent is bonded to the cationic or the anionic polymer through ionic bonds, wherein when the organosilane comprises at least a quaternary ammonium terminal group, it is bonded to the anionic polymer through ionic bonds, and wherein when the organosilane comprises at least a hydroxy or a carboxylate terminal group, it is bonded to the cationic polymer through ionic bonds. In one embodiment, optionally in combination with one or more features of the various embodiments described above or below, the encapsulation effciciency (EE) of the microcapsules of the invention is form 70to 100%, from 80to 100% or from 90 to 100%. The encapsulation efficiency (EE) may be measured as explained in the examples.
In one embodiment, optionally in combination with one or more features of the various embodiments described above or below, the weight amount of cationic polymer, in particular chitosan, is from 5 to 30%, more particularly from 10 to 25%, and even more particularly from 18 to 23%, with respect the total microcapsule shell weight.
In one embodiment, optionally in combination with one or more features of the various embodiments described above or below, the weight amount of anionic polymer, in particular alginate, is from 5 to 45%, more particularly from 10 to 30%, and even more particularly from 15 to 19%, with respect the total microcapsule shell weight.
In one embodiment, optionally in combination with one or more features of the various embodiments described above or below, the weight amount of silicate or silicate precursor, in particular sodium silicate or TEOS, is from 5 to 40%, more particularly from 10 to 30%, and even more particularly from 15 to 27%, with respect the total microcapsule shell weight.
In one embodiment, optionally in combination with one or more features of the various embodiments described above or below, the weight amount of the coupling agent, in particular APTES, is from 5 to 50%, more particularly from 15 to 45%, and even more particularly from 30 to 40%, with respect the total microcapsule shell weight.
In one embodiment, optionally in combination with one or more features of the various embodiments described above or below, the weight amount of the active ingredients is from 10 to 99.1%, more particularly from 75 to 99%, and even more particularly from 60 to 98%, with respect the total microcapsule weight.
According to the invention, the organic-inorganic core-shell microcapsules as previously defined further comprise a cross-linking agent which is a multivalent metallic cation as defined herein, particularly
in an amount from 0.1 to 55%, more particularly from 0.1 to 25%, in weight with respect to the total microcapsule shell weight.
In one embodiment, optionally in combination with one or more features of the various embodiments described above or below, the organic-inorganic core-shell microcapsule as previously defined further include one or more surfactants, more particularly, one or more non-ionic surfactants, in an amount from 0.1 to 5%, more particularly from 0.1 to 1%, in weight with respect to the total microcapsule weight.
In one embodiment, optionally in combination with one or more features of the various embodiments described above or below, the weight ratio between the cationic polymer, in particular chitosan, and the anionic polymer, in particular alginate, is from 0.5:1 to 1:0.5, more particularly is about 1:1.
In one embodiment, optionally in combination with one or more features of the various embodiments described above or below, the weight ratio between the coupling agent and the alkali metal silicate or silicate precursor is from 0.1:1 to 1:1, more particularly is about 1:1.
In another embodiment, optionally in combination with one or more features of the various embodiments described above or below, the weight ratio between the alkali metal silicate or silicate precursor and the coupling agent is from 0.1:3 to 1:3, more particularly is about 1:1.5.
In one embodiment, optionally in combination with one or more features of the various embodiments described above or below, the weight ratio between the cationic polymer, in particular chitosan, and the silicate or silicate precursor, in particular sodium silicate or TEOS, is from 0.3:1 to 0.95:1, more particularly is about 0.5:1 to 0.90 :1
In one embodiment, optionally in combination with one or more features of the various embodiments described above or below, the weight ratio between the anionic polymer, in particular alginate, and the silicate or silicate precursor, in particular sodium silicate or TEOS, is from 0.3:1 to 0.95:1, more particularly is about 0.5:1 to 0.75 :1.
In one embodiment, optionally in combination with one or more features of the various embodiments described

above or below, the weight ratio between the cationic polymer, in particular chitosan, and the coupling agent, in particular APTES, is from 0.2:1 to 0.95:1, more particularly is about 0.3:1 to 0.65:1 or from 0.25:1 to 0.5:1.

In one embodiment, optionally in combination with one or more features of the various embodiments described above or below, the weight ratio between the anionic polymer, in particular alginate, and the coupling agent, in particular APTES, is from 0.2:1 to 0.95:1, more particularly is about 0.3:1 to 0.65:1.

In another embodiment, optionally in combination with one or more features of the various embodiments described above or below, the weight ratio between the anionic polymer, in particular alginate, and the coupling agent, in particular APTES, is from 0.2:1 to 0.5:1, more particularly is about 0.3:1 to 0.6:1.

In one embodiment, optionally in combination with one or more features of the various embodiments described above or below, in the organic-inorganic core-shell microcapsule as previously defined, the weight amount of the shell is from 0.1 to 50%, more particularly from 0.1 to 35%, and even more particularly from 0.1 to 10%, with respect the total microcapsule weight, provided that the sum of the weight of the core and the weight of the shell is equal to 100%.

In one embodiment, optionally in combination with one or more features of the various embodiments described above or below, the shell of the microcapsules comprises or consists of an anionic polymer, in particular alginate; a cationic polymer, in particular chitosan; a silicate or silicate precursor; a coupling agent, and one or more cross-linking agents.

In one embodiment, optionally in combination with one or more features of the various embodiments described above or below, the core of the organic-inorganic core-shell microcapsules comprises one or more active ingredients, more particularly hydrophilic active ingredients, in liquid form, more particularly in the form of an aqueous solution.

In one embodiment, optionally in combination with one or more features of the various embodiments described above or below, the core of the microcapsules comprises one or more active ingredients, more particularly hydrophilic and lipophilic active ingredients, and one or more surfactants in liquid form, more particularly in the form of an emulsion, and even more particularly in the form of an oil-in-water emulsion.

According to the invention, the organic-inorganic core-shell microcapsules as previously defined have a particle size distribution D90 from 2 to 800 μm, particularly 5 to 500 μm, more particularly from 10 to 200 μm, even more particularly from 20 to 80 μm, and even more particularly from 15 to 45 μm. The term "D90" refers to the value of particle size distribution where at least 90% of the microparticles have a size lower than or equal to that value.

The real average size of the microcapsules can be calculated using a "Image-ProPlus 5" or Image J software and the OM micrographs of the microcapsules. The size distribution can be measured by means of a HELOS BR supplied by Sympatec GmbH System Partikel Technik equipped with a R1 cuvette and a Helium-Neon Laser 5mW max output at 632.8 nm.

An advantage of the microcapsules of the invention having a size equal or higher than 2 μm (i.e. in a micrometer scale) is that they are not able to cross biological barriers and therefore, there is no risk that the microcapsules are accumulated in the body when used for example for personal or animal care.

The hybrid core-shell microcapsule of the disclosure may be defined by its preparation process. Thus, it also forms part of the disclosure, an organic-inorganic core-shell microcapsule, wherein:

a) the shell forms a network which comprises:

i) an anionic polymer which comprises a plurality of carboxylate or sulfonate groups,
ii) a cationic polymer which comprises a plurality of quaternary ammonium groups,
iii) an alkali metal silicate or a silicate precursor, and
iv) a coupling agent which is an organosilane comprising at least a terminal group selected from a quaternary ammonium group, hydroxy, and carboxylate, and

b) the core comprises one or more active ingredients in liquid form; wherein the microcapsule is obtainable by a process, which comprises:

a) providing an aqueous solution comprising a polymer which comprises a plurality of carboxylic acid or sulfonic acid groups; an alkali metal silicate or a silicate precursor; a coupling agent which is an organosilane comprising at least a terminal group selected from amino, hydroxy, and carboxylic acid; and one or more active ingredients to obtain mixture A, wherein in solution the polymer is in anionic form comprising a plurality of carboxylate or sulfonate groups, and the terminal group of the coupling agent is in the form of a quaternary ammonium group, hydroxy, or carboxylate group;
b) dissolving a polymer which comprises a plurality of amino groups in an acidic aqueous solution to obtain solution B, wherein in solution the polymer is in cationic form comprising a plurality of quaternary ammonium groups; and
c) dropping mixture A onto solution B to obtain the microcapsules.

For the purposes of the invention, the expressions "obtainable", "obtained" and similar equivalent expressions are used interchangeably and, in any case, the expression "obtainable" encompasses the expression "obtained".
All the embodiments disclosed herein for the microcapsule and the preparation process apply also for the microcapsules obtainable by this process.

Preparation processes

The present invention also relates to a process for the preparation of the organic-inorganic core-shell microcapsules as defined above, which comprises:

a) providing an aqueous solution comprising a polymer which comprises a plurality of carboxylic acid or sulfonic acid groups; an alkali metal silicate or a silicate precursor; a coupling agent which is an organosilane comprising at least a terminal group selected from amino, hydroxy, and carboxylic acid; and one or more active ingredients to obtain mixture A, wherein in solution the polymer is in anionic form comprising a plurality of carboxylate or sulfonate groups, and the terminal group of the coupling agent is in the form of a quaternary ammonium group, hydroxy, or carboxylate group;
b) dissolving a polymer which comprises a plurality of amino groups in an acidic aqueous solution and adding a cross-linker to obtain solution B, wherein in solution the polymer is in cationic form comprising a plurality of quaternary ammonium groups; and
c) dropping mixture A onto solution B to obtain the microcapsules.

In one embodiment, optionally in combination with one or more features of the various embodiments described above or below, step a) is carried out under stirring e.g. at 300 rpm.
In one embodiment, optionally in combination with one or more features of the various embodiments described above or below, the concentration of anionic polymer is from 0.5 % to 2 wt%, more particularly about 1 wt%.
In one embodiment, optionally in combination with one or more features of the various embodiments described above or below, step a) comprises: a1) providing an aqueous solution of the anionic polymer, optionally one or more water-soluble active ingredients, a silicate or precursor of it, and the coupling agent; and a2) optionally adding one or more oil-soluble active ingredients, provided that at least an active ingredient is added.
In one embodiment, optionally in combination with one or more features of the various embodiments described above or below, step a) comprises: a1) providing an aqueous solution of the anionic polymer and optionally one or more water-soluble active ingredients; a2) optionally adding one or more oil-soluble active ingredients, a3) adding a silicate or precursor of it and the coupling agent, provided that at least an active ingredient is added.
In one embodiment, optionally in combination with one or more features of the various embodiments described above or below, step a) comprises: a1) providing an aqueous solution of the anionic polymer and optionally one or more water-soluble active ingredients; a2) adding a silicate or precursor of it; a3) adding the coupling agent; and a4) optionally adding one or more oil-soluble active ingredients, provided that at least an active ingredient is added.
In one embodiment, optionally in combination with one or more features of the various embodiments described above or below, step a) comprises: a1) providing an aqueous solution of the anionic polymer and optionally one or more water-soluble active ingredients; a2) adding a silicate or precursor of it; a3) adding the coupling agent; a3') adding a surfactant; and a4) adding one or more oil-soluble active ingredients. More particularly, the emulsion is formed by using an ultrasonic homogenizer, even more particularly using three cycles of ON/OFF pulsation (30 s/59 s) each with the amplitude of 70%.
Step b) dissolving a cationic polymer which comprises a plurality of quaternary ammonium groups in an acidic aqueous solution to obtain solution B.
In one embodiment, optionally in combination with one or more features of the various embodiments described above or below, the concentration of cationic polymer is from 0.2 wt% to 1.5wt%, more particularly about 0.5 wt%.
In one embodiment, optionally in combination with one or more features of the various embodiments described above or below, step b) is carried out at a pH between 2.5 to 4, more particularly at about 4.
According to the invention, optionally in combination with one or more features of the various embodiments described above or below, step b) further comprises the addition of a cross-linker.
In one embodiment, optionally in combination with one or more features of the various embodiments described above or below, step b) further comprises the addition of a surfactant.
In one embodiment, optionally in combination with one or more features of the various embodiments described above or below, the dropping of mixture A onto solution B step c) comprises using spray or nozzle technology, more particularly step c) is carried out under stirring.
In one embodiment, optionally in combination with one or more features of the various embodiments described above or below, the process for the preparation of the organic-inorganic core-shell microcapsule as defined above, comprises:

a) providing an aqueous solution comprising a polymer which comprises a plurality of carboxylic acid or sulfonic acid groups; an alkali metal silicate or a silicate precursor; a coupling agent which is an organosilane comprising at least a terminal group selected from amino, hydroxy, and carboxylic acid; and one or more active ingredients to obtain mixture A, wherein in solution the polymer is in anionic form comprising a plurality of carboxylate or sulfonate groups, and the terminal group of the coupling agent is in the form of a quaternary ammonium group, hydroxy, or carboxylate group;
b) dissolving a polymer which comprises a plurality of amino groups in an acidic aqueous solution, adding a cross-linker and optionally a surfactant, to obtain solution B, wherein in solution the polymer is in cationic form comprising a plurality of quaternary ammonium groups; and
c) dropping mixture A onto solution B to obtain the microcapsules.

It also forms part of the disclosure an organic-inorganic core-shell microcapsule, wherein:

a) the shell forms a network which comprises:

i) an anionic polymer which comprises a plurality of carboxylate or sulfonate groups,
ii) a cationic polymer which comprises a plurality of quaternary ammonium groups,
iii) an alkali metal silicate or a silicate precursor, and
iv) a coupling agent which is an organosilane comprising at least a terminal group selected from a quaternary ammonium group, hydroxy, and carboxylate, and

b) the core comprises one or more active ingredients in liquid form; wherein the microcapsule is obtainable by a process, which comprises:

a) providing an aqueous solution comprising a polymer which comprises a plurality of carboxylic acid or sulfonic acid groups; an alkali metal silicate or a silicate precursor; a coupling agent which is an organosilane comprising at least a terminal group selected from amino, hydroxy, and carboxylic acid; and one or more active ingredients to obtain mixture A, wherein in solution the polymer is in anionic form comprising a plurality of carboxylate or sulfonate groups, and the terminal group of the coupling agent is in the form of a quaternary ammonium group, hydroxy, or carboxylate group;
b) dissolving a polymer which comprises a plurality of amino groups in an acidic aqueous solution, optionally a cross-linker, and optionally a surfactant, to obtain solution B, wherein in solution the polymer is in cationic form comprising a plurality of quaternary ammonium groups; and
c) dropping mixture A onto solution B to obtain the microcapsules.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention.

## Examples

Chitosan from shrimp shells, practical grade, was purchased from Aldrich Chemistry; Alginic acid, sodium salt, was purchased from Acros Organics; Sodium silicate solution, reagent grade, was purchased from Sigma-Aldrich; Tetraethyl orthosilicate, reagent grade, was purchased from Sigma-Aldrich; Calcium chloride, anhydrous, granular, was purchased from Sigma-Aldrich; Magnesium chloride, anhydrous, was purchased from Sigma-Aldrich; Copper chloride, powder, was purchased from Sigma-Aldrich; (3-Aminopropyl)trimethoxysilane, 97%, was purchased from Alfa Aesar; TWEEN 80 was purchased from Sigma-Aldrich; Spearmint oil, Menta china oil and Citronella oil were provided by Creaciones Aromáticas Industriales S.A, Polyphenols and flavonoids extracts obtained from Cistus L.(cultivated in Turkey and distributed by Radix-Bis Sp. z o.o. (Rotmanka, Poland)) applying the solid-liquid extraction method described in Trojanowska et al. Chemical Engineering Research and Design 2019, 147, pp. 378-389.

Elemental analysis carried out by Energy-dispersive X-ray spectroscopy with Environmental Scanning Electron Microscope (ESEM) and RAMAN spectroscopy were performed to confirm the presence of silicate structures in the shell of the capsules. SEM-EDXS measurements were performed using a scanning electron microscope, a FEI Quanta 600 microscope equipped with a PentaFETx3 Link Dispersive Energy X-ray Spectroscopy EDXS system managed by IncaOxford.A Si(Li) detector with an ultra-thin window ATW2, with a resolution of 137 eV at 5 (Mn$K\alpha$1) was used. The spectral data were acquired at the working distance of 10.6 mm with an acceleration voltage (AV) of 20 kV.

Raman spectra were recorded using a Renishaw inVia Raman microscope assembled with diode laser emitting a

785 nm wavelength. The spatial resolution of the optical system obtained by using a 50x long working distance objective was better than 2 μm. The 300 mW laser power was used to excite the Raman spectra. The position of Raman peaks was calibrated before collecting the data using a Si wafer. The Raman spectra were measured in the 300-750 $cm^{-1}$ spectral range. The data processing i.e. baseline subtraction, determination of spectral parameters of Raman bands, such as wavenumber of the peak centre position or intensity, integral intensity were performed using the appropriate package of the Renishaw control software Wire 5.2.

Fourier transform infrared (FTIR) spectra of samples were obtained at room temperature with a FTIR spectrophotometer (Vertex 70, Bruker) with a resolution of 4 cm-1 and scanning speed of 2 mm $s^{-1}$, in absorbance mode. An attenuated total reflection (ATR) accessory with thermal control and a diamond crystal a Golden Gate heated single reflection diamond ATR from Specac-Teknokroma) was used to obtain FTIR spectra.

Optical Microscope Zeiss Axiovert 40C for transmitted light, with the incorporated Invenio3SII microscope camera was used for the characterization of emulsion and capsules morphology.

Encapsulation efficiency (EE) of microcapsules was measured by Gas Chromatograph (Agilent Technologies 7890A) with HP-5 column (0.25 nm x 30 m x 0.25 um). The determination of Free Perfume Composition was calculated after liquid-liquid extraction method as described in US Patent Application 20130039962. The free essential oil composition in the slurry via % liquid-liquid extraction and Gas Chromatographic Mass Spectrometric analysis was determined using equation for essential oil leakage index:

$$\text{Essential oil Leakage Index} = \frac{\text{Area essential oil Raw Material caps} \cdot \text{Area Internal Standard Solution ref} \cdot \text{Weight ref}}{\text{Area Internal Standard Solution caps} \cdot \text{Area essential oil Raw Material ref} \cdot \text{Weight caps}}$$

The relative response factor (RRF) was calculated:

$$RRF = \frac{m_P \cdot A_{IS}}{A_P \cdot m_{IS}}$$

wherein $m_P$ is the amount of essential oil, mis - the amount in grams of tonalid, $A_{IS}$ - the area of tonalid and $A_P$ is the area of the essential oil (sum of peaks).

The percentage of essential oil was calculated:

$$\%\ \text{essential oil} = \frac{A_{PE} \cdot RRF \cdot m_{ISE} \cdot 100}{A_{ISE} \cdot m_E}$$

wherein $m_{ISE}$ is the amount of tonalid in grams, $m_E$ is the amount of encapsulates composition (slurry) in grams, RRF is the relative response factor, $A_{PE}$ is the area of essential oil (sum of peaks) and $A_{ISE}$ the area of tonalid.

The encapsulation efficiency was calculated:

$$EE\ [\%] = 100 - \%\ \text{essential oil in the slurry}$$

Polyphenols and flavonoids content was measured in the slurry by using the methods described in Tsibranska et al. Food and bioproducts processing 2011, pp.273-280. Total flavonoids were determined using the following method: A volume of 0.5 mL $AlCl_3$ was added to 0.5 mL diluted sample. After one hour at room temperature the absorbance was measured at 420 nm. Three parallel measurements were performed.

The total phenolic content was determined spectrophotometrically according to the following procedure: A volume of 0.5 mL of Folin-Ciocalteu's reagent was added to a flask, containing 0.5 mL of the sample and 10 mL $dH_2O$. After 5 min 8 mL of 7.5% aqueous $Na_2CO_3$ solution was added to the mixture. The prepared samples were kept in dark for two hours and then the absorbance was measured at 765 nm with UV/Vis Spectrophotometer.

Example 1

Hybrid organic/inorganic shell biodegradable capsules containing an oil in water nanoemulsion core were

prepared by a coacervation method. The encapsulate nanoemulsion core contained active compounds both in aqueous and oil phases. Prior to the encapsulation process, aqueous extract of polyphenols and flavonoids was prepared applying a solid - liquid extraction process, in which 10 g of Cistus dry material was mixed with 150 g of water at room temperature for 24h. Then the obtained extract was passed through a paper filter. Then, the collected permeate was used to prepare 1 wt% of alginate solution. Next, 0.41 g of sodium silicate was added to 33.18 g of this 1 wt% sodium alginate solution containing extracted polyphenols and flavonoids, and the solution was mixed using a magnetic stirrer set up at 300 rpm for 30 minutes. Then, 0.60 g of coupling agent (3-aminopropyl)trimethoxysilane was added to the solution and it was additionally mixed for 5 minutes. Next, 0.23 g of Tween80 was added and the solution was mixed for 5 more minutes. Finally, 11.06 g of Spearmint oil was added and a final nanoemulsion was formed by using an ultrasonic homogenizer in an ice-bath to maintain the system a room temperature. This step involved three cycles of ON/OFF pulsation (30 s/59 s) each with the amplitude of 70% to get the oil in water nanoemulsion. The obtained solution was inserted into the spray gun.

Then separately, a second solution was prepared by dissolving 0.19 g of $CaCl_2$ and 0.27 g of Tween80 in 54.07 g of 0.5 wt% chitosan aqueous solution (pH=4) using a magnetic stirrer at 500 rpm during 10 minutes.

Finally, the spray gun was applied to create the drops of the nanoemulsion containing the active compounds in both phases. Encapsulation by the coacervation occurred when the emulsion drops were immersed into the bath of the second polymeric solution. During the spraying step the distance between the spray gun nozzle and the chitosan/$CaCl_2$ solution was of 15 cm, and the solution in the bath was mixed by a magnetic stirrer at 100 rpm. Afterwards, the formed slurry was mixed using a magnetic stirrer at 300 rpm for 24 hours.

In this example the weight amount of chitosan is within the range from 5 to 30%, the weight amount of alginate is within the range 5 to 45%, the weight amount of silicate is within the range 5 to 40%, the weight amount of APTES is within the range 5 to 50%, and the weight amount of cross-linker is within the range 1 to 25%, wherein the % are given with respect the total microcapsule shell weight. Further, the weight amount of the shell is within the range from 0.1 to 10%, and the weight amount of the core is within the range from 90 to 99.9%, with respect the total microcapsule weight.

The presence of silica (Si K$\alpha$) in the capsule was confirmed by an elemental analysis using an Energy Dispersive X-ray spectroscopy tool of an Environmental Scanning Electron Microscope (ESEM) at 1.7 KeV, as well as by RAMAN spectroscopy, presenting the peak at the wavenumber of 477 $cm^{-1}$ corresponding to the Si-O-Si bond (FIG. 7 and 8).

The amount of total polyphenols and flavonoids in the extract used for the microcapsules preparation was measured by means of UV-Vis spectroscopy. 3.67 mg of total polyphenols (as the gallic acid equivalent) and 12.52 mg of total flavonoids (as the quercetin equivalent) were applied for microcapsules preparation. The encapsulation efficiency (EE) of the microcapsules was measured by Gas Chromatography. The EE for those microcapsules was of 84.7%. The capsule size was in a range of 10-50 $\mu$m.

Example 2

The procedure was analogous to Example 1, except that MilliQ water was used to prepare the sodium alginate solution. FIG. 4 shows an OM image at magnification of 40X of the obtained microcapsules after being stored for 4 months at 22 °C. No leakage was observed. Furthermore, after storing the microcapsules 1 year at 22 °C the amount of the essential oil in the capsules shell only slightly decreased from 86$\pm$2% to 79$\pm$2%.

Example 3

The procedure was analogous to Example 2, except that for the emulsion preparation a Menta China essential oil was used. The mean diameter of microcapsules was of 24 $\mu$m as determined by measurements of particles size from OM micrographs, in the range D90: 15-45 $\mu$m.

Example 4

The procedure was analogous to Example 2, except that for the emulsion preparation Citronella essential oil was used.

Example 5

The procedure was analogous to Example 1, except that instead of 0.23 g, 0.02 g of Tween 80 was added to the emulsion. The encapsulation efficiency (EE) of the obtained microcapsules was 61.6% as measured by Gas Chromatography. FIG. 5 shows an OM image at magnification of 40X after being stored for 4 months at 22 °C.

Example 6

The procedure was analogous to Example 2, except that instead of $CaCl_2$, 0.19 g of $MgCl_2$ was added to the solution in the bath.

Example 7

The procedure was analogous to Example 1, except that instead of sodium silicate, 0.41 g of TEOS in 0.615 g of ethanol was added.

Example 8

The procedure was analogous to Example 7, except that instead of $MgCl_2$, 0.19 g of $CuCl_2$ was added to the solution in the bath.

Example 9

The procedure was analogous to Example 1, except that instead of 0.5 wt% chitosan aqueous solution (pH=4), 1.0 wt% chitosan aqueous solution (pH=4) was used for the preparation of the solution in the bath.

Comparative example 1

The procedure was analogous to Example 2, except that neither $CaCl_2$ nor (3-Aminopropyl)trimethoxysilane were added to the solution in the bath and emulsion, respectively. When no coupling agent was used, it was observed that the capsule shells were not formed and that the essential oil formed emulsion was present in the slurry.

Comparative Example 2

The procedure was analogous to Example 1, except that instead of 0.5 wt% chitosan aqueous solution (pH=4), MilliQ water was used. FIG. 6 shows an OM image at magnification of 40X of the obtained microcapsules after being stored for 4 months at 22 °C. As can be seen no well-defined capsules were obtained.

**Citation List**

- Guideline for testing of chemicals, Organisation for Economic Cooperation and Development (OECD), number 301 Adopted: 17.07.92 (https://www.oecd.org/chemicalsafety/risk-assessment/1948209.pdf)
- Patrocinio A.F. et al. "Enantioselective synthesis of alpha-hydroxysilanes by bioreduction of aroyltrimethylsilanes", J. Chem. Soc., Perkin Trans. 1, 1999, pp. 3133-3137
- Tachibana Y. et al., "Hydroxyl Terminated Hydrophilic Silanes", Silicon 2012, 4, pp. 167-174
- Feinle A. et al., "Stable carboxylic acid derivatized alkoxy silanes", Chem. Commun. 2015, 51, pp. 2339-2341
- Issa A. A. et al, "Kinetics of Alkoxysilanes and Organoalkoxysilanes Polymerization: A Review", Polymers 2019, 11, pp. 537
- Trojanowska et al., "Ultrasound-assisted extraction of biologically active compounds and their successive concentration by using membrane processes", Chemical Engineering Research and Design 2019, 147, pp. 378-389.
- US Patent Application 20130039962
- Tsibranska et al., "Extraction of biologically active compounds from Sideritis ssp. L.", Food and Bioproducts Processing 2011, Volume 89, Issue 4, pp. 273-280


**Claims**

**1.** Organic-inorganic core-shell microcapsules having a particle size distribution D90 from 2 to 800 μm, defined and measured as described in the description, wherein:

a) the shell of each microcapsule forms a network which comprises:

i) an anionic polymer which comprises a plurality of carboxylate or sulfonate groups,
ii) a cationic polymer which comprises a plurality of quaternary ammonium groups,
iii) an alkali metal silicate or a silicate precursor, and
iv) a coupling agent which is an organosilane comprising at least a terminal group selected from a quaternary ammonium group, hydroxy, and carboxylate, and
v) a cross-linking agent which is a multivalent metallic cation, and

b) the core of each microcapsule comprises one or more active ingredients in liquid form.

**2.** The organic-inorganic core-shell microcapsules according to claim 1, wherein the anionic polymer is selected form the group consisting of alginate, carrageenan, gellan gum, carboxyl methyl cellulose, hyaluronic acid, and a combination thereof.

**3.** The organic-inorganic core-shell microcapsules according to any of the claims 1-2, wherein the cationic polymer is selected form the group consisting of chitosan, N,O-carboxymethyl-chitosan, N,O-glycolic-chitosan, poly-L-lysine, and a combination thereof.

**4.** The organic-inorganic core-shell microcapsules according to any of the claims 1-3, wherein the anionic polymer is alginate, and the cationic polymer is chitosan.

**5.** The organic-inorganic core-shell microcapsules according to any of the claims 1-4, wherein the silicate precursor has the formula $Si(R^1)_m(R^2)_n$, wherein:

m and n are an integer from 1 to 3 provided that n+m is 4;
each $R^1$ is independently halogen, hydroxy or $-O(C_1-C_{12})$alkyl; and
each $R^2$ is independently selected from halogen, $-(C_1-C_{12})$alkyl, vinyl, allyl, phenyl, and $-O(C_1-C_{12})$alkyl; wherein $-(C_1-C_{12})$alkyl, and $-O(C_1-C_{12})$alkyl are unsubstituted or substituted with one or more substituents selected from the group consisting of halogen, hydroxy, glycicyl and methacryloyl.

**6.** The organic-inorganic core-shell microcapsules according to any of the claims 1-5, wherein the coupling agent is an organosilane of the formula $Si(R^3)_o(R^4)_p$, wherein:

o and p are an integer from 1 to 3 provided that o+p is 4;
each $R^3$ is independently halogen, hydroxy or $-O(C_1-C_{12})$alkyl; and
each $R^4$ has independently one of the formulas below:

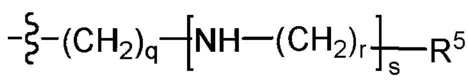

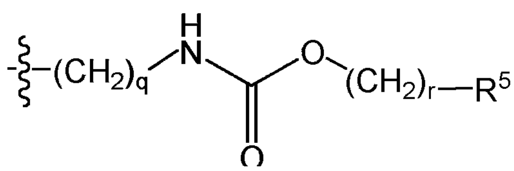

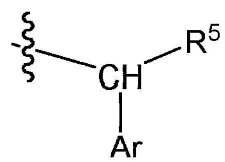

wherein q is an integer from 1 to 5, r is an integer from 1 to 5, s is an integer from 0 to 2, Ar is unsubstituted $(C_6-C_{12})$aryl or $(C_6-C_{12})$aryl substituted with one or more substituents selected from the group consisting of halogen, hydroxy, $-(C_1-C_3)$alkyl, $-(C_1-C_3)$alkyl substituted with one or more halogen atoms, $-O(C_1-C_3)$alkyl, and $-O(C_1-C_3)$alkyl substituted with one or more halogen atoms, and
$R^5$ is selected from the group consisting of $-NH_3^+$, $-NH_2^+R^6$, hydroxy and carboxylate, and

$R^6$ is hydrogen or $-(C_1-C_6)$alkyl.

**7.** The organic-inorganic core-shell microcapsules according to any of the claims 1-6, wherein the weight amount of cationic polymer with respect to the total microcapsule shell weight is from 5 to 30%.

**8.** The organic-inorganic core-shell microcapsules according to any of the claims 1-7, wherein the weight amount of anionic polymer with respect to the total microcapsule shell weight is from 5 to 45%.

**9.** The organic-inorganic core-shell microcapsules according to any of the claims 1-8, wherein the weight amount of silica precursor with respect to the total microcapsule shell weight is from 5 to 40%.

**10.** The organic-inorganic core-shell microcapsules according to any of the claims 1-9, wherein the weight amount of coupling agent with respect the total microcapsule shell weight is from 5 to 50%.

**11.** A composition comprising the organic-inorganic core-shell microcapsules as defined in any of the claims 1-10, and one or more excipients or carriers.

**12.** A process for the preparation of the organic-inorganic core-shell microcapsules as defined in any of the claims 1-10, which comprises:

a) providing an aqueous solution comprising a polymer which comprises a plurality of carboxylic acid or sulfonic acid groups; an alkali metal silicate or a silicate precursor; a coupling agent which is an organosilane comprising at least a terminal group selected from amino, hydroxy, and carboxylic acid; and one or more active ingredients to obtain mixture A, wherein in solution the polymer is in anionic form comprising a plurality of carboxylate or sulfonate groups, and the terminal group of the coupling agent is in the form of a quaternary ammonium group, hydroxy, or carboxylate group;
b) dissolving a polymer which comprises a plurality of amino groups in an acidic aqueous solution and adding a cross-linker to obtain solution B, wherein in solution the polymer is in cationic form comprising a plurality of quaternary ammonium groups; and
c) dropping mixture A onto solution B to obtain the microcapsules.

**13.** Use of the organic-inorganic core-shell microcapsules as defined in any of the claims 1-10, or alternatively of the composition as defined in claim 11 as a disinfectant.

**14.** Use of the organic-inorganic core-shell microcapsules as defined in any of the claims 1-10, or alternatively of the composition as defined in claim 11 for the delivery of active ingredients.

## Patentansprüche

**1.** Organisch-anorganische Kern-Hülle-Mikrokapseln mit einer Partikelgrößenverteilung D90 von 2 bis 800 μm, definiert und gemessen wie in der Beschreibung beschrieben, wobei:

a) die Hülle jeder Mikrokapsel ein Netzwerk bildet, das Folgendes umfasst:

i) ein anionisches Polymer, das eine Vielzahl von Carboxylat- oder Sulfonatgruppen umfasst,
ii) ein kationisches Polymer, das eine Vielzahl von quartären Ammoniumgruppen umfasst,
iii) ein Alkalimetallsilicat oder einen Silicatvorläufer und
iv) einen Haftvermittler, der ein Organosilan ist, das mindestens eine Endgruppe umfasst, die aus einer quartären Ammoniumgruppe, Hydroxy und Carboxylat ausgewählt ist, und
v) ein Vernetzungsmittel, welches ein mehrwertiges Metallkation ist, und

b) der Kern jeder Mikrokapsel einen oder mehrere Wirkstoffe in flüssiger Form umfasst.

**2.** Die organisch-anorganischen Kern-Hülle-Mikrokapseln nach Anspruch 1, wobei das anionische Polymer ausgewählt ist aus der Gruppe bestehend aus Alginat, Carrageen, Gellan, Carboxylmethylcellulose, Hyaluronsäure und einer Kombination davon.

3. Die organisch-anorganischen Kern-Hülle-Mikrokapseln nach einem der Ansprüche 1 bis 2, wobei das kationische Polymer ausgewählt ist aus der Gruppe bestehend aus Chitosan, N,O-Carboxymethyl-Chitosan, N,O-Glycol-Chitosan, Poly-L-Lysin und einer Kombination davon.

4. Die organisch-anorganische Kern-Hülle-Mikrokapseln nach einem der Ansprüche 1 bis 3, wobei das anionische Polymer Alginat ist und das kationische Polymer Chitosan ist.

5. Die organisch-anorganische Kern-Hülle-Mikrokapseln nach einem der Ansprüche 1 bis 4, wobei der Silicatvorläufer die Formel $Si(R^1)_m(R^2)_n$ hat, wobei:

m und n eine ganze Zahl von 1 bis 3 sind, unter der Voraussetzung, dass n + m = 4 ist;
jedes $R^1$ unabhängig voneinander Halogen, Hydroxy oder $-O(C_1-C_{12})$-Alkyl ist; und
jedes $R^2$ unabhängig voneinander ausgewählt ist aus Halogen, $-(C_1-C_{12})$-Alkyl, Vinyl, Allyl, Phenyl, und $-O(C_1-C_{12})$-Alkyl; wobei das $-(C_1-C_{12})$-Alkyl, und das $-O(C_1-C_{12})$-Alkyl unsubstituiert oder mit einem oder mehreren Substituenten substituiert sind, die ausgewählt sind aus der Gruppe bestehend aus Halogen, Hydroxy, Glycicyl und Methacryloyl.

6. Die organisch-anorganischen Kern-Hülle-Mikrokapseln nach einem der Ansprüche 1 bis 5, wobei der Haftvermittler ein Organosilan der Formel $Si(R^3)_o(R^4)_p$ ist, wobei:

o und p eine ganze Zahl von 1 bis 3 sind, unter der Voraussetzung, dass o + p = 4 ist;
jedes $R^3$ unabhängig voneinander Halogen, Hydroxy oder $-O(C_1-C_{12})$-Alkyl ist; und
jedes $R^4$ unabhängig voneinander eine der folgenden Formeln hat:

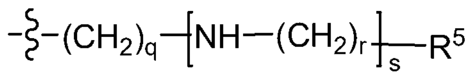

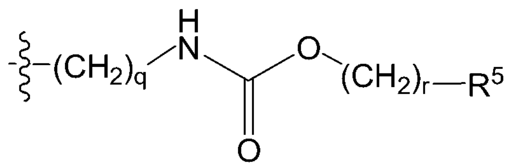

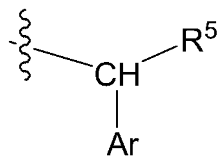

wobei q eine ganze Zahl von 1 bis 5 ist, r eine ganze Zahl von 1 bis 5 ist, s eine ganze Zahl von 0 bis 2 ist, Ar unsubstituiertes $(C_6-C_{12})$-Aryl oder $(C_6-C_{12})$-Aryl ist, das mit einem oder mehreren Substituenten substituiert ist, die ausgewählt sind aus der Gruppe bestehend aus Halogen, Hydroxy, $-(C_1-C_3)$-Alkyl, $-(C_1-C_3)$-Alkyl, das mit einem oder mehreren Halogenatomen substituiert ist, $-O(C_1-C_3)$-Alkyl, und $-O(C_1-C_3)$-Alkyl, das mit einem oder mehreren Halogenatomen substituiert ist, und
$R^5$ ausgewählt ist aus der Gruppe bestehend aus $-NH_3^+$, $-NH_2^+R^6$, Hydroxy und Carboxylat und $R^6$ Wasserstoff oder $-(C_1-C_6)$-Alkyl ist.

7. Die organisch-anorganischen Kern-Hülle-Mikrokapseln nach einem der Ansprüche 1 bis 6, wobei die Gewichtsmenge des kationischen Polymers in Bezug auf das Gesamtgewicht der Mikrokapselhülle von 5 bis 30 % beträgt.

8. Die organisch-anorganischen Kern-Hülle-Mikrokapseln nach einem der Ansprüche 1 bis 7, wobei die Gewichtsmenge des anionischen Polymers in Bezug auf das Gesamtgewicht der Mikrokapselhülle von 5 bis 45 % beträgt.

9. Die organisch-anorganischen Kern-Hülle-Mikrokapseln nach einem der Ansprüche 1 bis 8, wobei die Gewichtsmenge des Silicavorläufers in Bezug auf das Gesamtgewicht der Mikrokapselhülle von 5 bis 40 % beträgt.

10. Die organisch-anorganischen Kern-Hülle-Mikrokapseln nach einem der Ansprüche 1 bis 9, wobei die Gewichtsmenge des Haftvermittlers in Bezug auf das Gesamtgewicht der Mikrokapselhülle von 5 bis 50 % beträgt.

**11.** Eine Zusammensetzung umfassend die organisch-anorganischen Kern-Hülle-Mikrokapseln wie in einem der Ansprüche 1 bis 10 definiert, und einen oder mehrere Hilfsstoffe oder Trägersubstanzen.

**12.** Ein Verfahren zur Herstellung der organisch-anorganischen Kern-Hülle-Mikrokapseln wie in einem der Ansprüche 1 bis 10 definiert, welches Folgendes umfasst:

a) Bereitstellen einer wässrigen Lösung, umfassend ein Polymer, das eine Vielzahl von Carbonsäure- oder Sulfonsäuregruppen umfasst; ein Alkalimetallsilicat oder einen Silicatvorläufer; ein Haftvermittler, das ein Organosilan ist, das mindestens eine Endgruppe umfasst, die aus Amino, Hydroxy und Carbonsäure ausgewählt ist; und einen oder mehrere Wirkstoffe, um eine Mischung A zu erhalten, wobei das Polymer in Lösung in anionischer Form vorliegt und eine Vielzahl von Carboxylat- oder Sulfonatgruppen umfasst, und die Endgruppe des Haftvermittlers in Form einer quartären Ammoniumgruppe, Hydroxy- oder Carboxylatgruppe vorliegt;
b) Lösen eines Polymers, das eine Vielzahl von Aminogruppen umfasst, in einer sauren wässrigen Lösung und Zugeben eines Vernetzers, um eine Lösung B zu erhalten, wobei das Polymer in Lösung in kationischer Form vorliegt und eine Vielzahl von quartären Ammoniumgruppen umfasst; und
c) Tropfen der Mischung A auf die Lösung B, um die Mikrokapseln zu erhalten.

**13.** Verwendung der organisch-anorganischen Kern-Hülle-Mikrokapseln wie in einem der Ansprüche 1 bis 10 definiert, oder alternativ der Zusammensetzung wie in Anspruch 11 definiert als Desinfektionsmittel.

**14.** Verwendung der organisch-anorganischen Kern-Hülle-Mikrokapseln wie in einem der Ansprüche 1 bis 10 definiert, oder alternativ der Zusammensetzung wie in Anspruch 11 definiert für die Abgabe von Wirkstoffen.

## Revendications

**1.** Microcapsules à noyau-enveloppe organiques-inorganiques ayant une distribution de taille des particules D90 de 2 à 800 μm, définie et mesurée comme décrit dans la description, dans lesquelles :

a) l'enveloppe de chaque microcapsule forme un réseau qui comprend :

i) un polymère anionique qui comprend une pluralité de groupes carboxylate ou sulfonate,
ii) un polymère cationique qui comprend une pluralité de groupes ammonium quaternaire,
iii) un silicate de métal alcalin ou un précurseur de silicate, et
iv) un agent de couplage qui est un organosilane comprenant au moins un groupe terminal choisi parmi un groupe ammonium quaternaire, hydroxy et carboxylate, et
v) un agent de réticulation qui est un cation métallique multivalent, et

b) le noyau de chaque microcapsule comprend un ou plusieurs ingrédients actifs sous forme liquide.

**2.** Les microcapsules à noyau-enveloppe organiques-inorganiques selon la revendication 1, dans lesquelles le polymère anionique est choisi dans le groupe constitué par l'alginate, le carraghénane, la gomme gellane, la carboxyméthylcellulose, l'acide hyaluronique et une combinaison de ceux-ci.

**3.** Les microcapsules à noyau-enveloppe organiques-inorganiques selon l'une quelconque des revendications 1 à 2, dans lesquelles le polymère cationique est choisi dans le groupe constitué par le chitosane, le N,O-carboxyméthyl-chitosane, le N,O-glycolique-chitosane, la poly-L-lysine, et une combinaison de ceux-ci.

**4.** Les microcapsules à noyau-enveloppe organiques-inorganiques selon l'une quelconque des revendications 1 à 3, dans lesquelles le polymère anionique est l'alginate, et le polymère cationique est le chitosane.

**5.** Les microcapsules à noyau-enveloppe organiques-inorganiques selon l'une quelconque des revendications 1 à 4, dans lesquelles le précurseur de silicate a la formule $Si(R^1)_m(R^2)_n$, dans laquelle :

m et n sont un nombre entier de 1 à 3 à condition que n + m soit 4 ;
chaque $R^1$ est indépendamment halogène, hydroxy ou alkyle en $-O(C_1-C_{12})$ ; et

chaque $R^2$ est indépendamment choisi parmi halogène, alkyle en -($C_1$-$C_{12}$), vinyle, allyle, phenyle, et alkyle en -O($C_1$-$C_{12}$) ; où l'alkyle en -($C_1$-$C_{12}$), et l'alkyle en -O($C_1$-$C_{12}$) sont non substitués ou sont substitués avec un ou plusieurs substituants choisis dans le groupe constitué par l'halogène, l'hydroxy, le glycicyle et le méthacryloyle.

**6.** Les microcapsules à noyau-enveloppe organiques-inorganiques selon l'une quelconque des revendications 1 à 5, dans lesquelles l'agent de couplage est un organosilane de formule $Si(R^3)_o(R^4)_p$, dans laquelle :

o et p sont un nombre entier de 1 à 3 à condition que $\sigma$ + p soit 4 ;
chaque $R^3$ est indépendamment halogène, hydroxy ou alkyle en -O($C_1$-$C_{12}$) ; et
chaque $R^4$ a indépendamment l'une des formules ci-dessous :

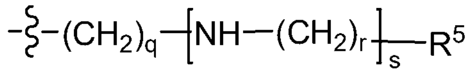

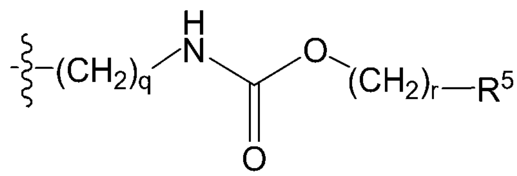

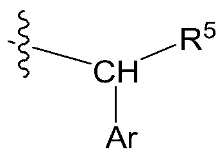

dans laquelle q est un nombre entier de 1 à 5, r est un nombre entier de 1 à 5, s est un nombre entier de 0 à 2, Ar est aryle en ($C_6$-$C_{12}$) ou aryle en ($C_6$-$C_{12}$) substitué avec un ou plusieurs substituants choisis dans le groupe constitué par halogène, hydroxy, alkyle en - ($C_1$-$C_3$), alkyle en -($C_1$-$C_3$) substitué avec un ou plusieurs atomes d'halogène, alkyle en - O($C_1$-$C_3$) et alkyle en -O($C_1$-$C_3$) substitué avec un ou plusieurs atomes d'halogène, et $R^5$ est choisi dans le groupe constitué par $-NH_3^+$, $-NH_2^+R^6$, hydroxy et carboxylate, et $R^6$ est hydrogène ou alkyle en -($C_1$-$C_6$).

**7.** Les microcapsules à noyau-enveloppe organiques-inorganiques selon l'une quelconque des revendications 1 à 6, dans lesquelles la quantité en poids de polymère cationique par rapport au poids total de l'enveloppe de microcapsule est de 5 à 30 %.

**8.** Les microcapsules à noyau-enveloppe organiques-inorganiques selon l'une quelconque des revendications 1 à 7, dans lesquelles la quantité en poids de polymère anionique par rapport au poids total de l'enveloppe de microcapsule est de 5 à 45 %.

**9.** Les microcapsules à noyau-enveloppe organiques-inorganiques selon l'une quelconque des revendications 1 à 8, dans lesquelles la quantité en poids de précurseur de silica par rapport au poids total de l'enveloppe de microcapsule est de 5 à 40 %.

**10.** Les microcapsules à noyau-enveloppe organiques-inorganiques selon l'une quelconque des revendications 1 à 9, dans lesquelles la quantité en poids d'agent de couplage par rapport au poids total de l'enveloppe de microcapsule est de 5 à 50 %.

**11.** Une composition comprenant les microcapsules à noyau-enveloppe organiques-inorganiques telles que définies dans l'une quelconque des revendications 1 à 10, et un ou plusieurs excipients ou véhicules.

**12.** Un procédé de préparation des microcapsules à noyau-enveloppe organiques-inorganiques telles que définies dans l'une quelconque des revendications 1 à 10, qui comprend :

a) fournir une solution aqueuse comprenant un polymère qui comprend une pluralité de groupes acide carboxylique ou acide sulfonique ; un silicate de métal alcalin ou un précurseur de silicate ; un agent de couplage

qui est un organosilane comprenant au moins un groupe terminal choisi parmi les groupes amino, hydroxy et acide carboxylique ; et un ou plusieurs ingrédients actifs pour obtenir un mélange A, dans lequel en solution le polymère est sous forme anionique comprenant une pluralité de groupes carboxylate ou sulfonate, et le groupe terminal de l'agent de couplage est sous la forme d'un groupe ammonium quaternaire, d'un groupe hydroxy ou d'un groupe carboxylate ;
b) dissoudre un polymère qui comprend une pluralité de groupes amino dans une solution aqueuse acide et ajouter un agent de réticulation pour obtenir une solution B, dans laquelle en solution le polymère est sous forme cationique comprenant une pluralité de groupes ammonium quaternaire ; et
c) verser le mélange A sur la solution B pour obtenir les microcapsules.

**13.** Utilisation des microcapsules à noyau-enveloppe organiques-inorganiques telles que définies dans l'une quelconque des revendications 1 à 10, ou alternativement de la composition telle que définie dans la revendication 11 en tant que désinfectant.

**14.** Utilisation des microcapsules à noyau-enveloppe organiques-inorganiques telles que définies dans l'une quelconque des revendications 1 à 10, ou alternativement de la composition telle que définie dans la revendication 11 pour la délivrance d'ingrédients actifs.

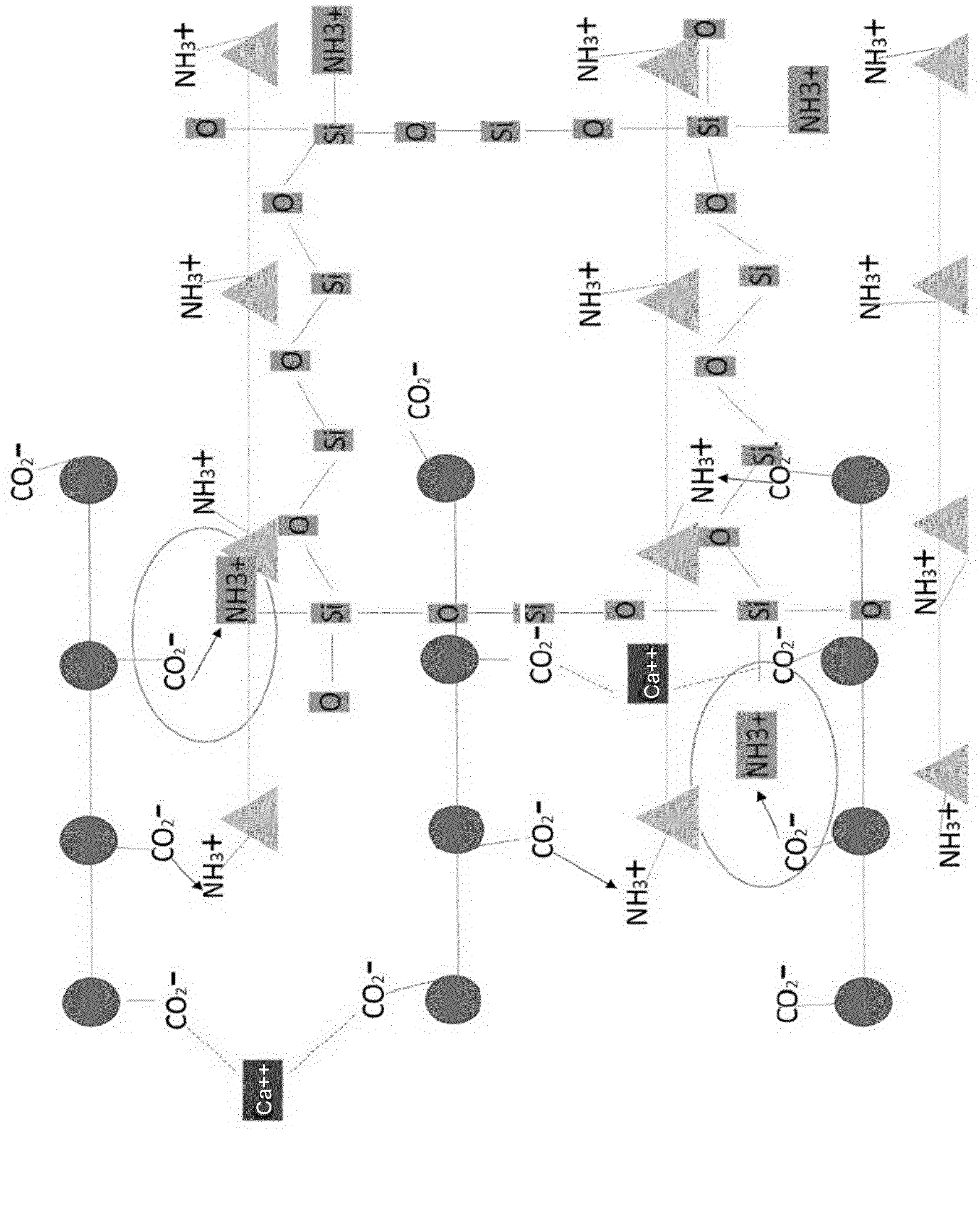

FIG. 1

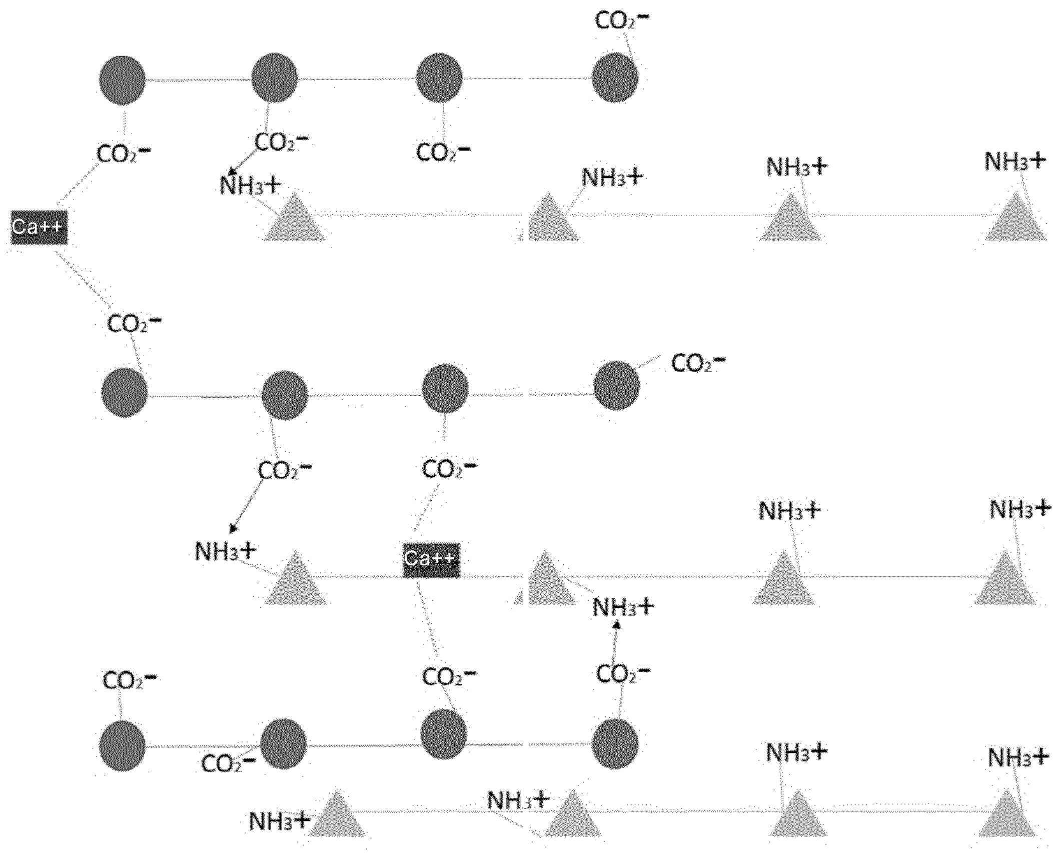

FIG. 2

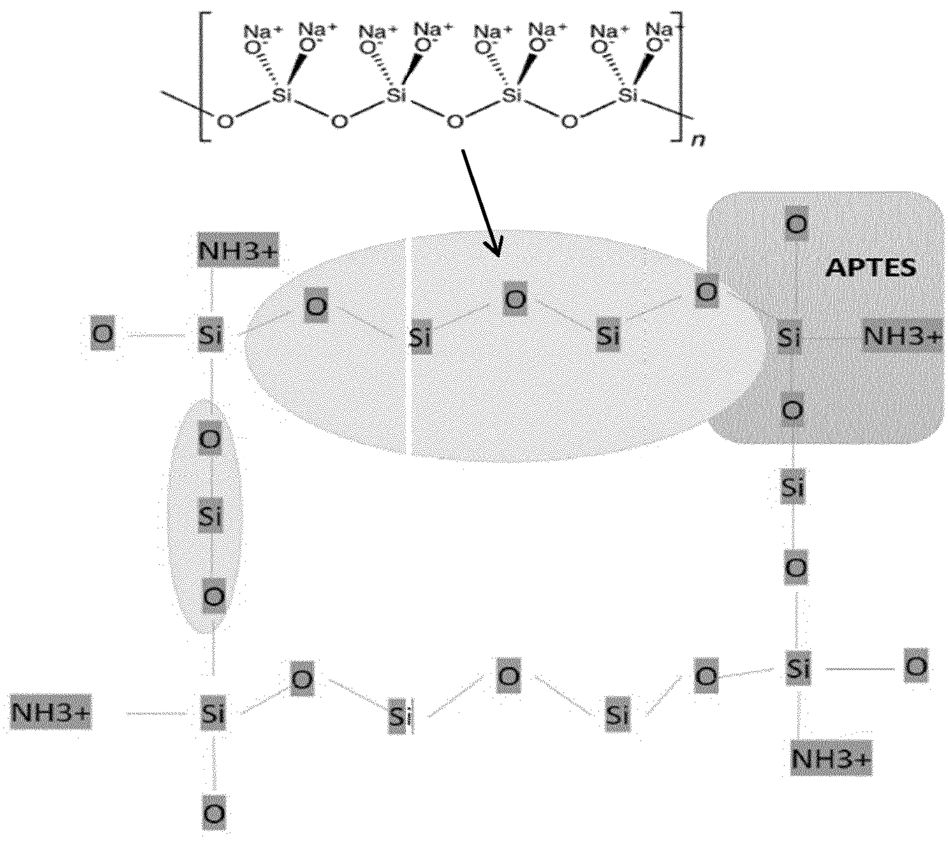

FIG. 3

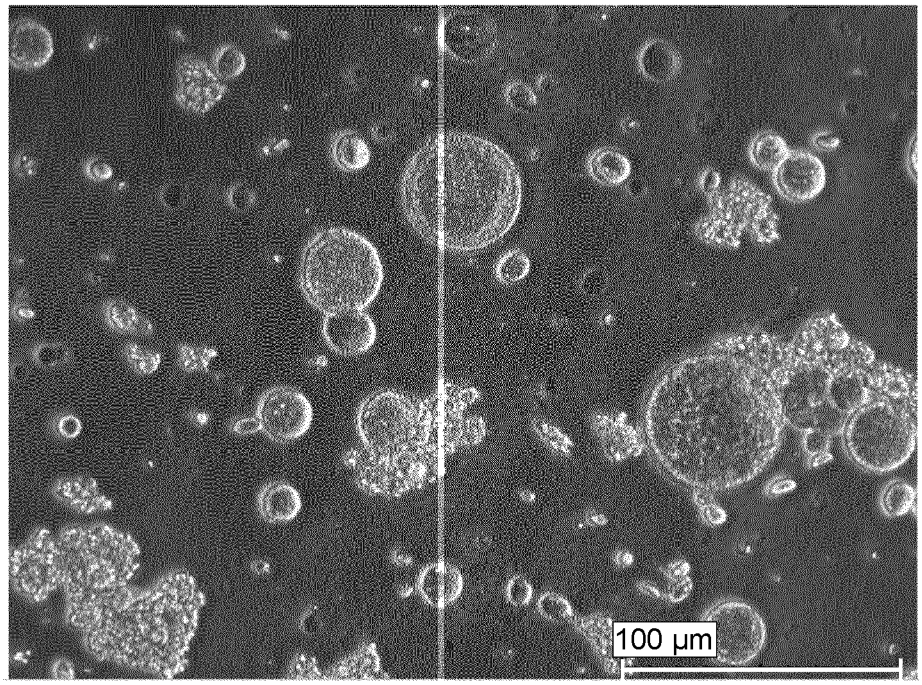


FIG. 4

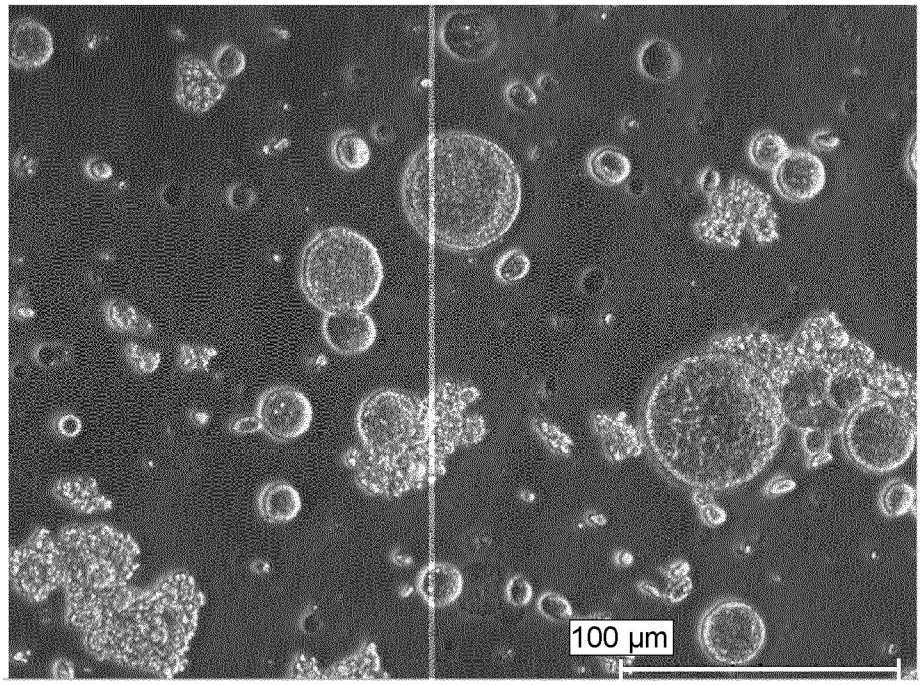


FIG. 5

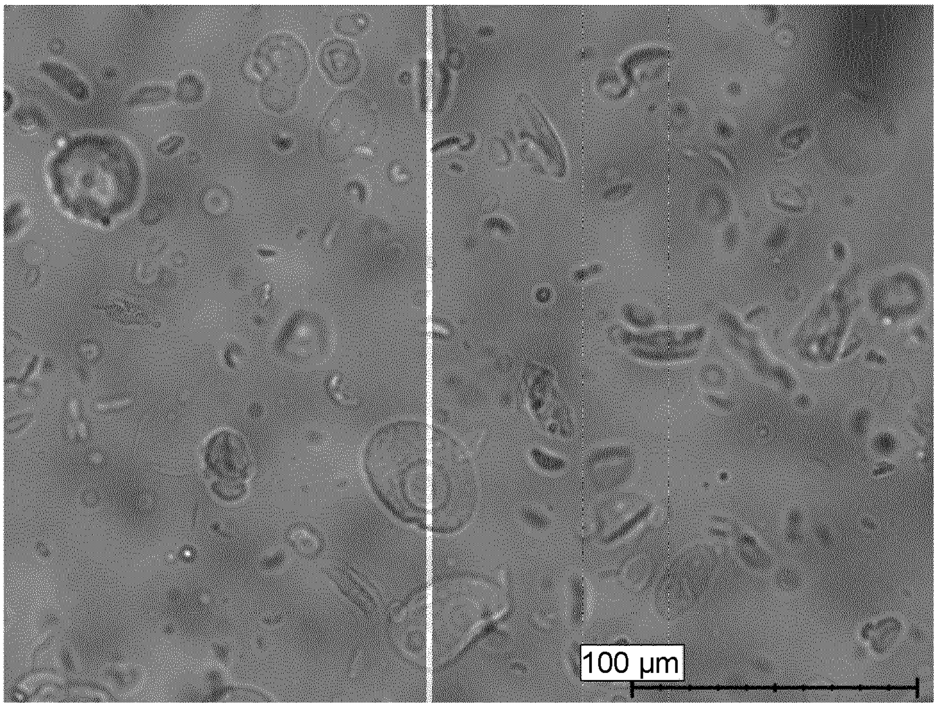
100 µm

FIG. 6

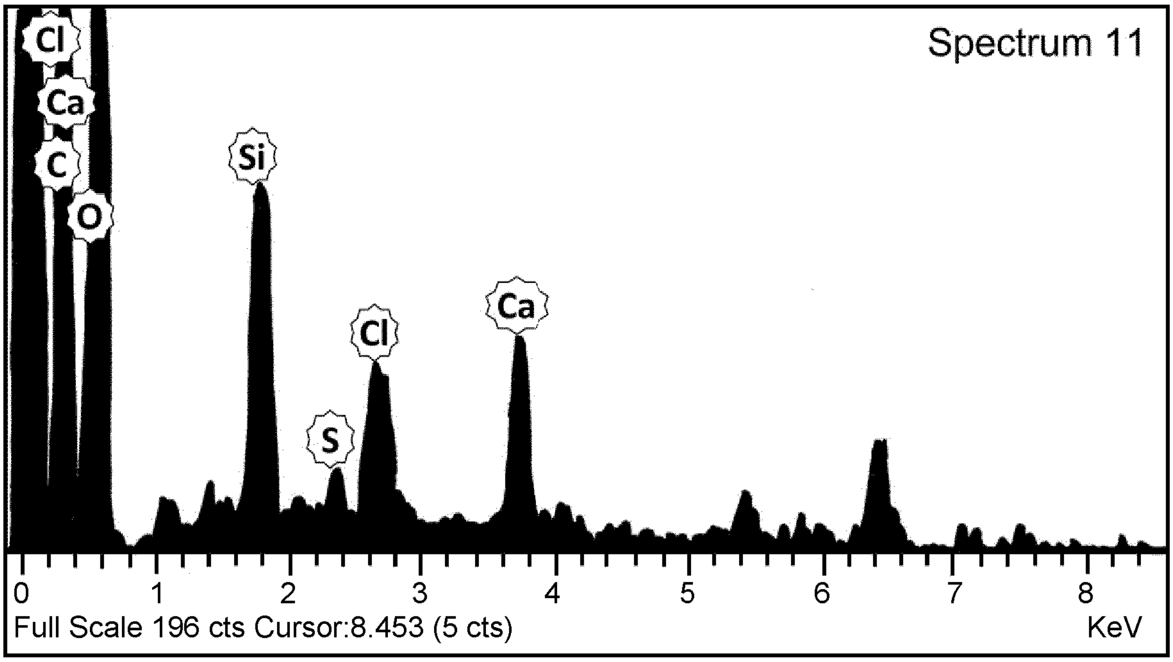
Spectrum 11
Cl
Ca
C
O
Si
S
Cl
Ca
0
1
2
3
4
5
6
7
8
Full Scale 196 cts Cursor:8.453 (5 cts)
KeV

FIG. 7

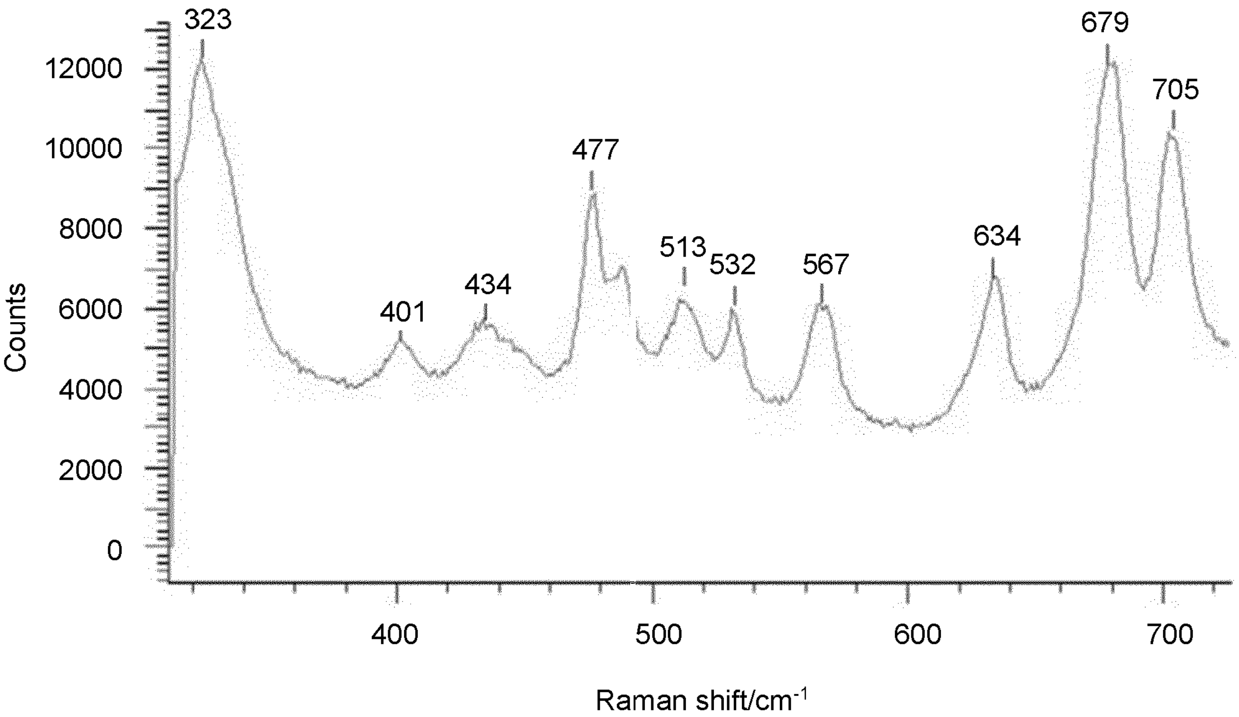
323
679
705
477
513
532
567
634
401
434
12000
10000
8000
6000
4000
2000
0
Counts
400
500
600
700
Raman shift/cm-1

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104825421
- WO 2017015885 A
- US 20130039962 A


### Non-patent literature cited in the description


- **PATROCINIO A.F. et al.** *J. Chem. Soc., Perkin Trans*, 1999, vol. 1, 3133-3137
- **TACHIBANA Y. et al.** *Silicon*, 2012, vol. 4, 167-174
- **FEINLE A et al.** *Chem. Commun.*, 2015, vol. 51, 2339-2341
- **ISSA A. A. et al.** *Polymers*, 2019, vol. 11, 537
- **TROJANOWSKA et al.** *Chemical Engineering Research and Design*, 2019, vol. 147, 378-389
- **TSIBRANSKA et al.** *Food and bioproducts processing*, 2011, 273-280
- **PATROCINIO A.F. et al.** Enantioselective synthesis of alpha-hydroxysilanes by bioreduction of aroyltrimethylsilanes. *, J. Chem. Soc., Perkin Trans*, 1999, vol. 1, 3133-3137
- **TACHIBANA Y. et al.** Hydroxyl Terminated Hydrophilic Silanes. *Silicon*, 2012, vol. 4, 167-174
- **FEINLE A. et al.** Stable carboxylic acid derivatized alkoxy silanes. *Chem. Commun.*, 2015, vol. 51, 2339-2341
- **ISSA A. A. et al.** Kinetics of Alkoxysilanes and Organoalkoxysilanes Polymerization: A Review. *Polymers*, 2019, vol. 11, 537
- **TROJANOWSKA et al.** Ultrasound-assisted extraction of biologically active compounds and their successive concentration by using membrane processes. *Chemical Engineering*, 2019, vol. 147, 378-389
- **TSIBRANSKA et al.** Extraction of biologically active compounds from Sideritis ssp. L.. *Food and Bioproducts Processing*, 2011, vol. 89 (4), 273-280